# EUROPEAN PATENT SPECIFICATION

(11) **EP 4 025 114 B1**
(45) Date of publication and mention of the grant of the patent: **30.07.2025**
(21) Application number: 20767791.5
(22) Date of filing: 04.09.2020
(51) Int. Cl.: A61B 3/024, A61B 3/10, A61B 3/12, A61B 3/00, G16H 10/60, G16H 30/20, G16H 30/40, G16H 40/63, G16H 50/20, G16H 50/70

(54) **MACHINE LEARNING METHODS FOR CREATING STRUCTURE-DERIVED VISUAL FIELD PRIORS**
MASCHINENLERNVERFAHREN ZUR ERZEUGUNG VON STRUKTURABGELEITETEN SICHTFELDPRIOREN
PROCÉDÉS D'APPRENTISSAGE MACHINE POUR CRÉER DES ANTÉCÉDENTS DE CHAMP VISUEL DÉRIVÉS DE STRUCTURES

(30) Priority: 06.09.2019 US 201962897025 P
(43) Date of publication of application: 13.07.2022
(73) Proprietor: Carl Zeiss Meditec, Inc., Dublin, CA 94568 (US); Carl Zeiss Meditec AG, 07745 Jena (DE)
(72) Inventor: LEE, Gary, Dublin, CA 94568 (US); FLANAGAN, John G, Berkeley, CA 94708 (US)
(74) Representative: Nieten, Christoph
(86) International application number: PCT/EP2020/074766
(87) International publication number: WO 2021/043980

(56) References cited:
- EP-A1- 2 449 957
- WO-A1-2018/083853
- ZHIHUI GUO ET AL: "Optical Coherence Tomography Analysis Based Prediction of Humphrey 24-2 Visual Field Thresholds in Patients With Glaucoma", INVESTIGATIVE OPTHALMOLOGY & VISUAL SCIENCE, vol. 58, no. 10, 10 August 2017 (2017-08-10), pages 3975 - 3985, XP055749472, ISSN: 1552-5783, DOI: 10.1167/iovs.17-21832

## Description

### FIELD OF INVENTION

The present invention is generally directed to the field of visual field testing. More specifically, it is directed to a system and method for optimizing a field test for improved accuracy, improved repeatability, reduced overall test time, and for suggesting/identifying new locations in the visual field to test.

### BACKGROUND

Glaucoma is one of the leading causes of blindness in the world with 44.7 Million people with open-angle glaucoma world-wide and projected to reach 58.6 million worldwide in 2020. While the use of optical coherence tomography (OCT) and optical coherence tomography angiography (OCTA) are becoming more common in the management of glaucoma, the analysis of visual fields (VFs) remains the clinical gold standard of diagnosing and staging glaucoma, as well as for monitoring functional vision loss over time.

A visual field test is a method of measuring an individual's entire scope of vision, e.g., their central and peripheral (side) vision. Visual field testing is a way to map the visual fields of each eye individually and can detect blind spots (scotomas) as well as more subtle areas of dim vision.

A campimeter, or "perimeter," is a dedicated machine/device/system that applies a visual field test to a patient. There are different types of perimeters and different types of visual field tests, but all visual field tests are subjective examinations. A patient must therefore be able to understand the testing instructions, fully cooperate, and complete the entire test while alert in order to provide useful information. Complicating this is the reality that a visual field test can take a relatively long time, which may fatigue a patient and compromise test results.

A common visual field test type, or algorithm, is the standard automated perimetry (SAP) test, which determines how dim a light can be and still be perceived (e.g., the threshold) at various points in an individual eye's visual field. Various algorithms have been developed to determine this threshold for different, individual test points in a single visual field. The Swedish interactive thresholding algorithm (SITA) may be combined with the SAP test to determine visual fields more efficiently, for example, when used with a Humphrey Field Analyzer (HFA) from ZEISS^{®}. The SITA algorithm optimizes the determination of perimetry thresholds by continuously estimating what the expected threshold is based on the patient's age and neighboring thresholds. For example, depending on a patient's response to a first stimulus, the intensity of each subsequent stimulus presentation is modified. This iterative procedure is repeated until the likely threshold measurement error is reduced to below a predetermined level, with 1 or more reversals typically occurring at every test location. In this manner, it can reduce the time necessary to acquire a visual field, decrease patient fatigue, and thereby increase reliability. Improvements to SITA have resulted in SITA Fast and SITA Faster algorithms, which can reduce test times even further. Similar to the SITA test strategy for the HFA, the tendency-oriented perimeter (TOP) algorithm was developed for use with the Octopus^{™} perimeter as an alternative to its lengthy staircase threshold procedures. Nonetheless, visual field tests typically still take several minutes to perform for each eye, even with state of the art test strategies, such as the various versions of SITA. Test times also tend to increase with more damaged or glaucomatous visual fields.

Overall, test strategies with shorter test times may help increase the frequency of visual field testing in glaucoma management, bringing clinical glaucoma care more in line with current recommendations of professional organizations. Shorter test times are generally preferred by patients, minimize the effects of patient fatigue leading to more reliable test results, and reduce the cost of testing.

It is an object of the present invention to reduce the overall test time of a visual field test.

It is another object of the present invention to reduce thresholding visual field test durations (e.g., the time needed for a patient to reach his/her minimum visible light threshold for an individual test point) with minimal or no loss of clinical accuracy.

It is a further object of the present invention to provide a system and method for improved predictions of a patient's expected threshold for individual test points.

It is still another object of the present invention to make use of structural and/or functional characteristics of a patient's eye, obtained by use of a different ophthalmic examination modality, to aid in the reducing of thresholding visual field test durations.
EP 2 449 957 A1 describes a method to find starting intensity values for a VF test based on biometric measurements (retinal layer thickness).
Document ZHIHUI GUO ET AL: "Optical Coherence Tomography Analysis Based Prediction of Humphrey 24-2 Visual Field Thresholds in Patients With Glaucoma", INVESTIGATIVE OPTHALMOLOGY & VISUAL SCIENCE, vol. 58, no. 10, 10 August 2017, pages 3975-3985 and WO 2018/083853 describe similar methods using machine learning techniques.

### SUMMARY OF INVENTION

The above objects are met in a method/system for customizing visual field tests comprising the steps/features as defined in claims 1 and 9, respectively. The method/system may have multiple elements, including: a data system for selecting a visual field test for a patient, where the selected visual field test has one or more test points of definable light intensity. A biometric (e.g., structural or functional) measurement of a retina of the patient is obtained, or otherwise accessed, such as from an electronic medical record (EMR). The biometric measurement may be collected by use of an optical coherence tomography (OCT) system, OCT angiography system, fundus imager, or other ophthalmic examination system modality for collecting physical/empirical ophthalmic data. For example, the biometric measurement may be based, at least in part, on an image of the retina, which may include 3D, or depth-resolved, data. A computing system or network, such as one embodying a machine learning architecture (e.g., an artificial intelligence system and/or neural network system), may be used to predict a respective threshold sensitivity value for one or more select test points of the selected visual field test based at least in part on the obtained biometric measurement(s). Each predicted threshold sensitivity value may include a light intensity measure that the patient is expected to discern with a predefined success rate (e.g., a 50% success rate), and/or an area measure (e.g., illuminated point/shape/region of specific size) that the patient is expected to discern at a given brightness level, and/or a combination of both. A visual test system may use the predicted threshold sensitivity values as "priors," e.g., inputs to the selected visual field test (which may use the priors to optimize the patient's FA test), and/or as starting intensity/area values for the one or more select test points when applying the selected visual field test to the patient. By using starting intensity values close to the patient's final test results, the patient can reach his/her threshold values more quickly, resulting in an overall shorter test duration.

Additionally or alternatively, predicted threshold sensitive values may be used as synthesized VF priors in place of, or in addition to, true VF prior in a VF forecast system. The VF forecast system may use the synthetized VF priors (and optionally any available true VF priors) to forecast a future visual field for a patient.

Other objects and attainments together with a fuller understanding of the invention will become apparent and appreciated by referring to the following description and claims taken in conjunction with the accompanying drawings.

Several publications may be cited or referred to herein to facilitate the understanding of the present invention. All publications cited or referred to herein, are hereby incorporated herein in their entirety by reference.

The embodiments disclosed herein are only examples, and the scope of this disclosure is not limited to them.

### BRIEF DESCRIPTION OF THE DRAWINGS

In the drawings wherein like reference symbols/characters refer to like parts:
FIG. 1 provides an overview of a system for customizing visual field tests in accord with the present invention.
FIG. 2 shows an example of the training of a neural network NN-1 in accord with the present invention.
FIG. 3 illustrates an example operation of the trained neural network NN-1 of FIG. 2 either with live data input after training or with test data input during an evaluation phase of a training session.
FIG. 4 illustrates an alternate training architecture that links multiple NN stages, Stg1 and Stg2, each including its own neural network in a modular neural network configuration.
FIG. 5 illustrates how having a patient's visual field test history may be helpful in predicting (forecasting) the patient's present, or future, visual field threshold value(s) for a given test point.
FIG. 6 illustrates a visual field forecast system in accord with the present invention.
FIGs. 7A and 7B are plots of (derived) OCT-Estimated Threshold vs (true) VF Threshold for machine learning models based on a random forest approach (FIG. 7a) and a neural network approach (FIG. 7b).
FIG. 8 illustrates Table 1, which shows that overall Mean Absolute Errors (MAEs) for *ZEST-RF* and *ZEST-CNN* were statistically equivalent to *ZEST* (p<0.001).
FIG. 9 illustrates an example of a visual field test instrument (perimeter) for testing a patient's visual field.
FIG. 10 illustrates an example of a slit scanning ophthalmic system for imaging a fundus.
FIG. 11 illustrates a generalized frequency domain optical coherence tomography system used to collect 3-D image data of the eye suitable for use with the present invention.
FIG. 12 shows an example of an *en face* vasculature image.
FIG. 13 illustrates an example of a multilayer perceptron (MLP) neural network.
FIG. 14 shows a simplified neural network consisting of an input layer, a hidden layer, and an output layer.
FIG. 15 illustrates an example convolutional neural network architecture.
FIG. 16 illustrates an example U-Net architecture.
FIG. 17 illustrates an example computer system (or computing device or computer).

### DESCRIPTION OF THE PREFERRED EMBODIMENTS

In a typical visual field (VF) test, a patient is presented with a number of test points distributed (e.g., sequentially) over a visual field, and asked to discern the appearance of individual test points. The size and/or light intensity of individual test points may be adjusted until the patient is able to identify the appearance of an individual test point with at predefined success rate, such as 50%. This final size and/or intensity of a test point defines that test point's threshold value, which may be the basis for a visual sensitivity measure incorporated into the visual field test's results. If the initial size and/or intensity of a test point is far from its final threshold value, many adjustment iterations may be needed before "thresholding" (e.g., reaching the patient's threshold value for that specific test point), leading to a longer test time. Thus, a goal of efficient thresholding strategies is to select initial size and/or intensity values for individual test points that are close to their final threshold values for a specific patient, and thereby lead to shortened visual field test times.

Efficient thresholding strategies have been pushing the limits of threshold testing. One approach toward improving thresholding is using visual field "priors," or prior information (e.g., historical data or statistically models derived from historical data) used to estimate a patient's future VF test performance. Bayesian strategies by default incorporate the idea of prior information or data that are updated with each stimulus presentation (e.g., test point) and response. The Swedish Interactive Thresholding Algorithm (SITA) and the Zippy Estimation by Sequential Testing (ZEST) perimetric algorithms are examples of strategies that use Bayesian prior techniques. A discussion of SITA may be found in "SITA Fast, A New Rapid Perimetric Threshold Test, Description of Methods and Evaluation in Patients with Manifest and Suspect Glaucoma," by Boel Bengtsson et al., Acta Ophthalmologica Scandinavica, 1998: 76: 431-437, and in "A New SITA Perimetric Threshold Testing Algorithm: Construction and a Multicenter Clinical Study," by Anders Hejil et al., American Journal of Ophthalmology, Vol. 198, Feb 2019, Pages 154-165. Similarly, a discussion of ZEST may be found in "Targeted Spatial Sampling Using GOANNA Improves Detection of Visual Field Progression," by Chong et al., Ophthalmic Physiol Opt, 2015, Mar, 35(2):155-69. All of these references are herein incorporated in their entirety by reference. The priors (e.g., previously collected data and/or population-derived data) are often based on uniform values (often supra-threshold/bright), related to age-matched data, or even derive from previous visual fields of the same patient. Some limitations of using these priors are that uniform or age-matched data are not individualized to a given patient, meaning extra stimuli at a given location might be required. Visual field priors of the same patient are possible, but may often not be unavailable (e.g., first visit by patient) or be out of date due to VF tests not being administered as often as other tests. That is, because VF tests are subjective and take more time than other more typical ophthalmic tests, such as structural/imaging tests, VF tests might not be administered at their recommended intervals.

A newer approach toward facilitating perimetry is to construct structure-derived visual fields, which may include one or more of derived visual fields, derived visual sensitivity measures, and derived priors based on one or more sources of quantifiable data, such as ophthalmic images, patient-specific physiological characteristics/measures, medical condition(s), medical treatment(s), (visual) evoked potential tests, and/or other vision-related testing. Structural imaging, such as optical coherence tomography (OCT) imaging, has been used to estimate (e.g., derive) visual fields, which have typically been positioned as "replacement" fields for functional VF testing (e.g., for use in place of standard/functional visual field testing). Because structural data is often more reproducible than functional VF data, structural data may provide the benefit of more reproducible, derived visual fields. A limitation of previous structure-derived visual fields is that they are typically generated with custom mathematical models that are often tied to a specific instrument, as is discussed in "Relationships of Retinal Structure and Humphrey 24-2 Visual Field Thresholds in Patients with Glaucoma," by Bogunovic et al., Invest. Ophthalmol. Vis. Sci., 2015; 56(1): 259-271, herein incorporated in its entirety by reference. This use of custom mathematical models tied to specific instruments limits the utility of structure-derived visual fields. Another obstacle to previous structure-derived visual fields is that standard (functional) visual fields are still considered the gold standard for evaluating visual function. Consequently, functional visual fields may be more trusted by the general clinician than estimated visual fields derived from structural priors.

In evoked potential (EP) tests, or evoked response (ER) tests, electrodes are used to record an electrical potential response from a specific part of a patient's nervous system, typically the brain, following presentation of a stimulus (sensory stimulation), such as through light, sound, or touch. For example, an evoked potential test may measure the time it takes for the brain to respond to a sensory stimulation. In a visual evoked potential (VEP) test, electrodes may be placed on the patient's scalp while the patient sits in front of a screen and watches a changing light pattern (e.g., first with one eye, and then with the other). A VEP test may record each eye's response to the changing pattern. For example, the patient may be asked to gaze at a checkerboard pattern on the screen while the colors of the squares alternate at a predefined frequency and/or in a predefined pattern, and the VEP test records which changes the patient was able to perceive based on the patient's evoked potential response.

However, it is believed that structural priors have not been used to facilitate the construction/administering of standard, functional visual fields. Herein is proposed a method, system, and/or workflow that generates accurate (true/functional) visual fields with reduced test times in a novel way.

The present invention combines the use of ophthalmic imaging/examining systems (and/or their output results) with a visual field testing system (e.g., perimeter) to optimize a functional visual field test (e.g., optimize its starting points, e.g., the initial light intensity and/or size of test points of the functional visual field test). For example, the optimized starting points may be estimated/predicted to be close to a patient's expected thresholding (e.g., final) value for a given test point. In this manner, the number of (intensity and/or size) iterative adjustments for a given test point to reach the patient's threshold value is reduced, leading to a reduced overall test time. A discussion of a typical visual test system and typical (functional) visual field test, in general, is provided below in section "Visual Field Test System".

Various types of ophthalmic imaging/examining systems are known in the art, such as fundus imagers, OCT systems, and OCT angiography (OCTA) systems. Fundus imagers may take two-dimensional (2D) images of the surface of the retina, or other parts of the eye. Various structural measurements/observations may be made from fundus images. OCT and OCTA enable noninvasive, depth-resolved (e.g., A-scan), volumetric (e.g., C-scan) and 2D (e.g., *en face* or cross-sectional/B-scan) visualization of retinal vasculature. OCT may provide structural images of vasculature whereas OCTA may provide functional images (e.g., blood flow) of vasculature. For example, OCTA may image vascular flow by using the motion of flowing blood as an intrinsic contrast. These types of ophthalmic imaging systems are discussed below in section "Fundus Imaging System" and in section "Optical Coherence Tomography (OCT) Imaging System." Unless otherwise stated, aspects of the present invention(s) may apply to any, or all, such ophthalmic imaging systems. For example, the methods/systems presented herein for optimizing thresholding (e.g., optimizing the starting values of test points in, and/or providing synthesized "priors" for, a functional visual field test) may incorporate structural and/or functional (e.g., motion) ophthalmic information (biometrics measurements) extracted from an eye, and this ophthalmic information (biometric measurements) may be obtained by use of a fundus imager, OCT system, and/or OCTA system.

Some embodiments of the present invention leverage existing Bayesian type of strategies and follow-up with adding in synthesized/derived "prior" (e.g., synthesized visual fields) that are derived from structural and/or functional ophthalmic data/imaging (e.g., fundus image, OCT scan/image, OCTA scan/image, patient-specific physiological characteristics/measures, medical condition(s), medical treatment(s), (visual) evoked potential tests, and/or other vision-related testing) in place of true VF priors (e.g., prior functional VF test results taken by use of a perimeter). These prior synthesized visual fields may be derived using machine learning (ML) techniques, such as deep learning (DL) and/or artificial intelligence (AI) methods. That is, unlike prior approaches that use true VF priors to attempt to accelerate functional VF testing, the present approach proposes using a synthesized VF prior that is determined from structural (such as OCT and fundus imaging) and/or functional (such as OCTA imaging) ophthalmic information, herein collectively referred to as "biometric" and/or "physical characteristic" (measure/measurement) derived priors. An advantage of this approach is that biometric derived priors may be more repeatable (e.g., have less variability) and may often be less onerous to obtain for a subject (i.e. could be derived quickly at the same clinic visit of a patient prior to the patient receiving a traditional functional visual field test) than generating multiple (true) prior visual fields to establish a VF history for the subject. Additionally, the biometric derived priors may be created using methods of Artificial Intelligence (AI), Machine Learning (ML), and/or Deep Learning (DL).

It is to be understood that there are various different types of machine learning models known in the art, such as supervised learning, unsupervised learning, semi-supervised learning, reinforcement learning, etc. Although aspects of the present discussion provide examples using specific machine learning models, such as DL and AI, it is to be understood that other types of machine learning models, singularly or in combination, may be used with the present invention. For example, one or more of Nearest Neighbor, Naive Bayes, Decision Trees, Linear Regression, Support Vector Machines (SVM), and Neural Networks may be used to implement a supervised learning model in accord with the present invention.

By using machine learning techniques to derive biometric derived priors, the present invention not only has the potential to generate more robust and reproducible input (synthesized/derived) visual fields (VFs), but also takes advantage of some features intrinsic to those methods that may help provide better a understanding of an ophthalmic biometric in relation to a VF function model (e.g., identify relationships between observed biometric measurements and VF tests). By using biometric (e.g., image) data that is usually collected as part of a standard clinical workflow to create structural priors (e.g., biometric derived priors) in lieu of a true visual field prior (which may not be available from previous visits or is less reproducible) as input to a fast VF testing strategy (which may have no other available VF prior data), such as SITA, it is estimated that the present approach can potentially reduce current threshold VF test time by up to 30% in glaucomatous eyes. That is, the present invention pushes the limits of threshold testing beyond what can be achieved using modern thresholding strategy types alone, such as SITA and its variants, which may be reaching their optimization limits. For example, the present approach may expand these limited by introducing biometric measurements as additional sources of prior information for optimizing functional VF testing.

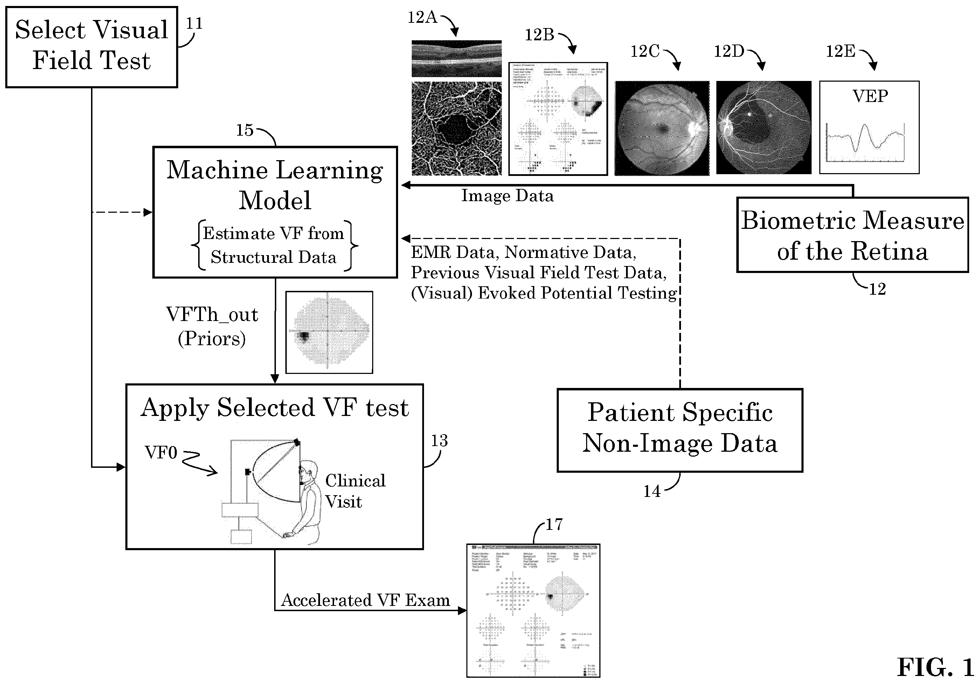

**FIG. 1** provides an overview of a system for customizing/optimizing traditional (functional) visual field tests in accord with the present invention. The system may include a graphical user interface (not shown) and electronic processor(s) to facilitate various processing steps. For example, a user/technician may start by specifying/selecting a specific VF test, block 11. The present system may be configured to function with any type of visual field test selected for use with a given perimeter (VF tester) VF0. The selected visual field test may be any of a static automated perimetry test, kinetic perimetry test, frequency doubling perimetry test, or other known perimetry test type using thresholding (e.g., visual sensitive measures) to determine a patient's visual field. Examples of known VF tests include standard automated perimetry (SAP), short-wavelength automated perimetry (SWAP), frequency doubling technology (FDT), Swedish Interactive Thresholding Algorithm (SITA), SITA Fast, SITA Faster, tendency-oriented perimeter (TOP), objective perimetry (VEP, multifocal electroretinography ERG/PERF, pupillometry, etc), etc. Irrespective of the type of VF test selected, the selected visual field test will generally have one or more test points of definable light intensities. Some VF tests also provide points of definable size(s) (e.g., area) at a given light intensity. An objective of the present system is to determine the threshold value (e.g., the ending intensity and/or size) of individual test points for a specific patient to whom a selected VF test is to be administered.

The present system obtains one or more biometric measurement (e.g., a physical characteristic measure), such as of the retina of the patient, (optionally including prior functional tests of the patient) to whom the visual test is to be administered, as indicated by block 12, to construct structure-derived visual fields. The biometric measurement may be based on an image of the retina obtained using any of multiple imaging modalities and/or images (e.g., photocopies, bitmap/raster/vector or other digital images, print-outs, etc.) of previous patient tests. For example, the imaging modality may be grayscale, color, infrared, retinal layer thickness map, fundus photography, optical coherence tomography (OCT), Doppler OCT, OCT angiography, and/or fluorescein angiography. The biometric measurement 12 may be extracted from (e.g., be based on) or include the entirety (or portion) of one or more OCT/OCTA image 12A, prior visual field test result 12B (or the main sensitivity values of the prior visual field test), fundus image 12C, fluorescence angiography (FA) image(s) 12D, VEP 12E, or other imaging modality or retinal/vision measuring technique/device. The biometric measurement may be obtained by use of an ophthalmic test system (e.g., an OCT system or fundus imager, not shown) directly on the patient at the time of the patient's visit to a clinic, or may be accessed from a data store of the patient's medical records, such as from an electronic medical record (EMR). Examples of the biometric measure may include one or more A-scans, B-scans, C-scans, or *en face* image obtained by use of an OCT/OCTA system. The biometric measure may include the shape, size, color, and/or relative position of individual ophthalmic structures, such as the optic nerve head (OHN), fovea, retinal thickness, and thickness measure of individual retina layer(s). Other examples of biometric measures may include blood flow measures and/or tissue motion measures at specific regions of the retina, regions of discoloration from an expected norm, regions of vascular conversion (e.g., their size, locations, and/or number), exudate formation (e.g., their size, locations, and/or number), large vessel count, small vessel count, identification of specific structures, some of which may be indicative of (e.g., associated with) pathology. For example, exudate-associated derangements are lesions that have been associated with certain types of "wet" age-related macular degeneration (AMD). The biometric measure may further include a comparison of the relative measures of different physiological features, such as the distance(s) between (and/or relative orientations/positionings of) specific structures and/or comparative size ratio(s) of specific structures.

The obtained biometric measure(s) may be submitted to a machine learning model 15, which may be embodied within one or more computing systems (e.g. electronic processors). It is to be understood that individual retinal images (e.g., OCT/OCTA, fundus, and/or FA images) may be submitted to machine model 15 as one or more biometric measure, and machine model 15 may extract individual biometric sub-measures from the submitted image(s), as needed. Optionally, the machine model 15 may also receive as input information regarding the specific VF test algorithm selected to be administered to the patient. For example, machine model 15 may be informed of the type of VF test that is to be administered to the patient, which may enable it to better cater its construction of a suitable biometric derived prior. Machine learning model 15 may determine (e.g., predict/synthesize/derive) a respective threshold value (e.g., visual sensitivity value) for one or more select test points of the selected VF test type based at least in part on its received biometric measurement(s). Each threshold sensitivity value may be based on a light intensity measure and/or point size measure for an individual VF test point that the patient is expected to discern with a predefined success rate (e.g., a 50% success rate). That is, machine learning model 15 outputs synthesized VF thresholds (e.g. VFTh_out), which may constitute one or more VF priors, e.g., a collection of numerical data (illustratively shown as a derived VF test output 10), and which may be used in conjunctions with the selected functional VF test administered to the patient, as indicated by block 13. Consequently, the present system results in an accelerated functional VF exam 17 (e.g., a VF exam of shorted time duration).

Optionally, the individual threshold sensitivity value(s) VFTh_out may be further based on additional, non-structural or image, patient related data, such as may be accessed from an EMR, as indicated by block 14. For example, determination of the threshold sensitivity value for the one or more select test points of the selected visual field test may be further based on patient-age specific normative data associated with the specific imaging device(es) (e.g., OCT and/or fundus imager) from which one or more of the biometric measure(s) were obtained. The prediction of the threshold sensitivity value(s) may also be based on non-structural patient-specific data (e.g., physiological data not extracted from the input retinal image(s) of block 12), such as one or more of the patient's age, ethnic group, and medical history. The determination of the threshold sensitivity values may also be based on prior patient-specific functional tests, such as prior VF test results and/or prior (visual) evoked potential test data.

To reiterate, the thus determined (e.g., predicted/derived) visual sensitivity value(s) VFTh_out may be submitted to the perimeter 13, which may base its starting VF test point values (e.g., intensity and/or size input priors) for the corresponding one or more select test point(s) (or otherwise optimize its VF test) when applying the selected functional VF test to the patient. That is, the derived sensitivities VFTh_out may be modified in the construction of priors. For example, the chosen VF test may start using input priors having an offset (e.g., higher or lower intensity) from the derived sensitivities VFTh_out.

Alternatively, or in addition, the determined, or estimated, threshold sensitivity value(s) may be used as VF priors and/or be used to determine a prediction of the patient's visual field that may be used for diagnostic/clinical interpretation or structure-function analyses. For example, the patient's predicted visual field may be used as part of a clinical decision support (CDS) system, which provides clinicians, staff, patients or other individuals with knowledge and person-specific information, intelligently filtered or presented at appropriate times, to enhance health care. The present system may be incorporated as an additional tool in a CDS system to enhance decision-making in the clinical workflow. For example, the present system may provide computerized alerts and reminders to care providers and patients and provide clinical guidelines, condition-specific order sets (e.g., recommendation for a visual field test or other medical test), focused patient data reports and summaries, documentation templates, diagnostic support, and contextually relevant reference information. For example, a current, derived sensitivities VFTh_out may compared with one or more previous derived sensitivity results and/or true visual field test results (e.g., from prior doctor visits), and issue a warning flag/message when the current, derived sensitivities VFTh_out indicate that the patient's visual field may be changing beyond a predefined range and/or a predefined area and/or a predefined rate of change. The warning flag/message may indicate that the patient should be scheduled for a true visual field test.

Machine learning model 15 may be based on one or more of linear regression, logistic regression, decision tree, support vector machine, naive Bayes, k-nearest neighbors, k-means, random forest, dimensionality reduction, gradient boosting, and neural network. Generally, a machine learning model is a computing system that can be trained to perform a specific function or functions, and selection of a specific model may depend on the type of problem being addressed. For example, a support vector machine (SVM) is a machine learning, linear model for classification and regression problems, and may be used to solve linear and non-linear problems. The idea of an SVM is to create a line or hyperplane that separates data into classes. More formally, an SVM defines one or more hyperplanes in a multi-dimensional space, where the hyperplanes are used for classification, regression, outlier detection, etc. Essentially, an SVM model is a representation of labeled training examples as points in multi-dimensional space, mapped so that the labeled training examples of different categories are divided by hyperplanes, which may be thought of as decision boundaries separating the different categories. When a new test input sample is submitted to the SVM model, the test input is mapped into the same space and a prediction is made regarding what category it belongs to based on which side of a decision boundary (hyperplane) the test input lies.

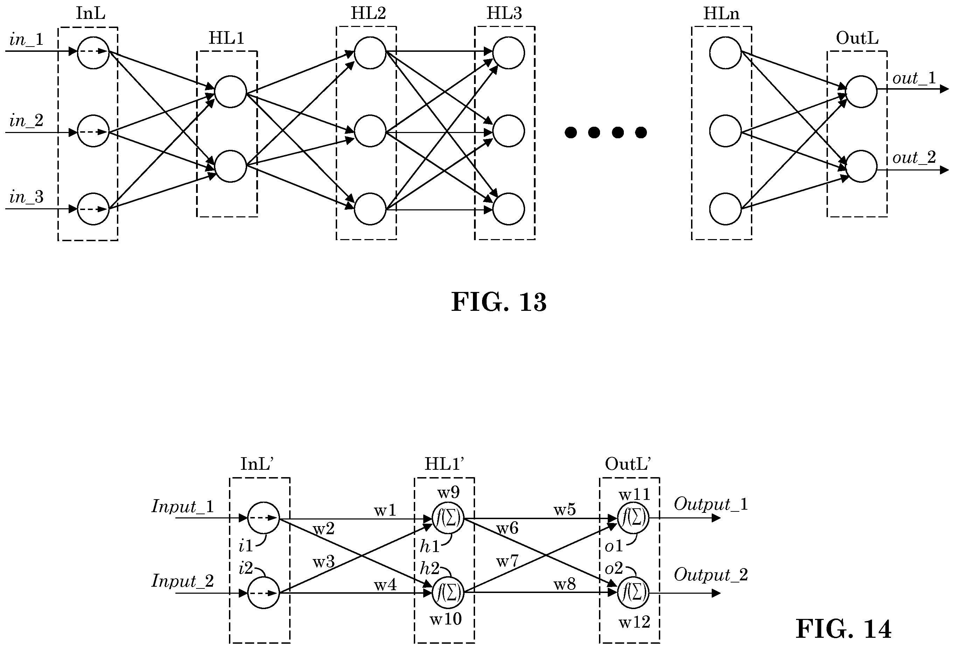

In a preferred implementation of the present invention, however, machine learning model 15 is realized, at least in part, within a computing system that includes/embodies a trained neural network, which may be based on deep learning. Various examples of neural networks are discussed below with reference to FIGS. 13 to 16, any of which, singularly or in combination, may be used with the present invention.

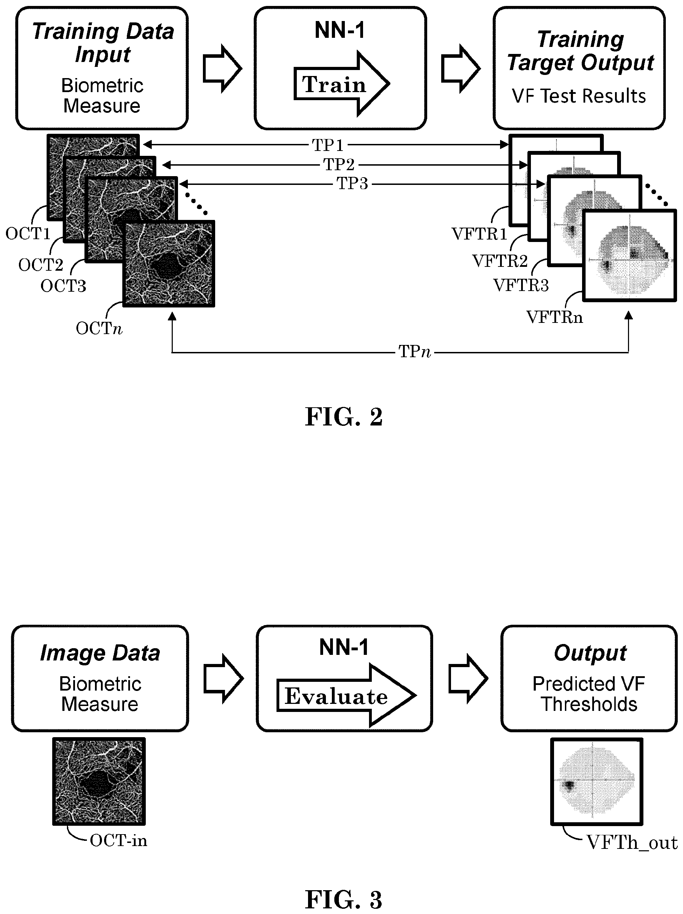

For illustration purposes, FIG. 2 shows an example of the training of a neural network NN-1 in accord with the present invention. For ease of discussion, each training set is shown to consist of training pairs TP1 to TPn, and in the present example, each training pair may include OCT-based image/scan OCT1 to OCTn (e.g., OCT angiography data and/or structural OCT data) as a training input samples paired with corresponding, labeled visual field test result data VFTR1 to VFTRn collected from the same "test patient" from which the training input images were obtained, and preferably on approximately the same date on which the training input images were collected. However, as is discussed above, in addition to, or in place of the OCT-based images, the training (data) input samples may include fundus images, fluorescein angiography images, (visual) evoked potential tests and other objective perimetry results (multifocal electroretinography ERG/PERF, pupillometry, etc.), individual retinal structural measurements, previously diagnosed pathologies (e.g., medical condition(s), medical treatment(s), and/or other medical records), physical feature(s) of the test patient (e.g., age, ethnic group, medical history), normative structural data of the demography of the test patient (retinal nerve fiber layer (RNFL) thickness and a ganglion cell-inner plexiform layer (GCIPL) thickness), normative functional data of the demography of the test patient (e.g., standardized initialization parameter of the specific visual field test), etc. The training (data) inputs may further include prior visual field test results (e.g., true or previously synthesized/derived functional VT test results / visual sensitivity measures) and dates on which they were taken, so to help identify tendencies in the rate of change in visual field results associated with specific characteristics (e.g., pathologies) of the test patient. It is to be understood that these prior visual field test results for training input may be based on SAP and/or they could be based on objective perimetry (VEP, multifocal ERG/PERF, pupillometry, etc.). For ease of illustration, the training input OCT-based data is shown as depth-encoded *en face* slab/images, but it is to be understood that the training input OCT data may be volume data, B-scans, or A-scans. In the present example, neural network NN-1 is trained to determine (or derive) VF priors, e.g., perimetry thresholds (e.g., intensity and/or size threshold values of individual perimetry test points of a given visual field test type), and so its visual field training target outputs VFTR1 to VFTRn are illustratively shown as labeled, true, functional visual field test results (e.g., darkened and light squares and/or numeral perimetry threshold results of individual test points distributed along a test visual field). In the present example, the neural network NN-1 is trained to extract perimetry threshold data from full OCT-based image information, and so the training input in each training pair is shown to include full scan information OCT1 to OCTn. Optionally, data augmentation methods may be used to increase the size of the training data set, such as by dividing each test input data (OCT1 to OCTn) into data segments (or image/scan patches) of smaller size, where the patches may be of similar or different sizes. Generally, a larger training set size provides better training results.

**FIG. 3** illustrates an example operation of the trained neural network NN-1 of FIG. 2 either with live data input after training is finished or with test data input during an evaluation phase of a training session. The trained neural network NN-1 may include one or more of a fully-connected neural network, convolutional neural network, feedforward neural network, recurrent neural network, modular neural network, and U-Net, as discussed more fully below. The present neural network NN-1 may receive obtained image data (e.g., a live image, such as from an OCT system or fundus imager, or access previously collected images, such as from the patient's medical records, which may be stored remotely) as input OCT-in (the input may optionally also specify the type of visual field test that is to be administered to a patient if multiple VF test types are supported), and predict (e.g. determine/synthesize/generate) a corresponding visual field threshold output VFTh_out having predicted threshold value(s) for one or more test points of the specified visual field test type. As shown in FIG. 1, output VFTh_out may be submitted to block 13 for administering a functional VF test to a patient. It is noted that input image OCT-in is not an image used in training, or an image derived from any image used in training. That is, image data (e.g., OCT-in) not seen before by the network NN-1 is selected for the testing/evaluation/operation phase. Optionally, in operation, network NN-1 does not receive as input any previous true (functional) visual field test results of the patient.

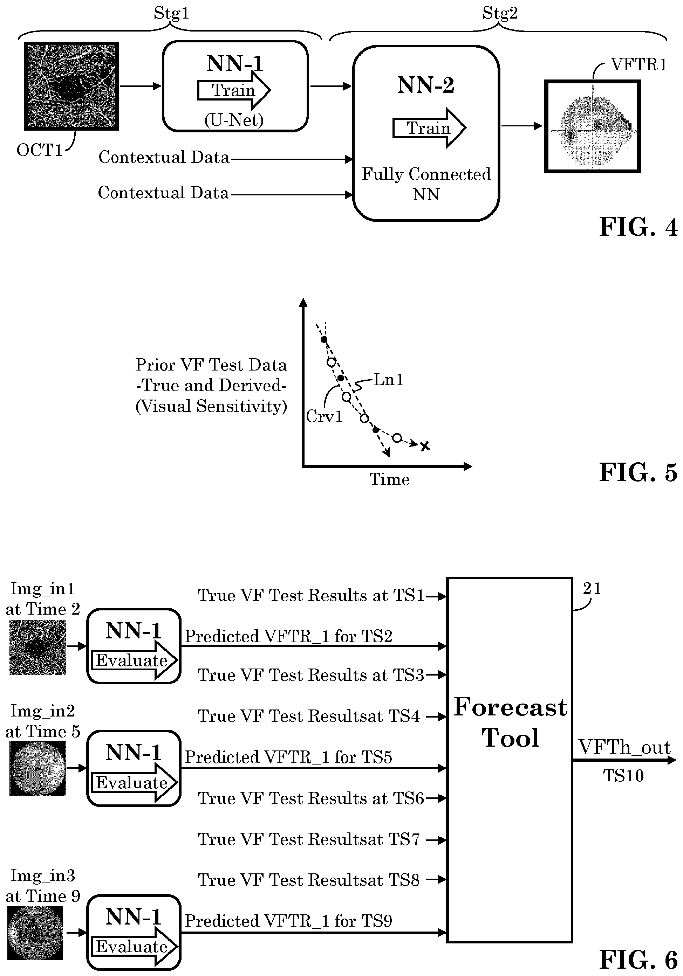

**FIG. 4** illustrates an alternate training architecture according to the invention that links multiple NN stages, Stg1 and Stg2, each including its own neural network in a modular neural network configuration. The first stage Stg1 of the present architecture is similar to that of FIG. 2, and may consists of a neural network optimized for processing images, such as a convolutional neural network and/or U-Net. All elements in FIG. 4 similar to those of FIG. 2 have similar reference characters and are discussed above. In the present example, the output from first stage Stg1 feeds into a second neural network NN-2, which may be optimized to process individual data units (as opposed to images) and may consists of, for example, a fully-connected neural network, feedforward neural network, and/or recurrent neural network. The inputs to the second stage Stg2 may exclude images, and include individual data sets (e.g., contextual data), such as normative data, patient medical records data, individual biometric measurements, previous (true or synthesized) visual field threshold results, etc. In operation (e.g., after training), the predicted VF thresholds from the architecture of FIG. 4 (not shown) could be submitted to the perimeter 13 of FIG. 1 to administer a functional visual field test using the predicted VF thresholds as starting test point values and/or priors.

It is noted that taking into consideration previous visual field test results may be helpful in identifying trends in a patient's changing visual field, which may lead to more accurate predictions. However, because heretofore visual field tests have been time-consuming and not always been administered at prescribed (e.g., regular) intervals, there may be gaps in the visual field test results of a patient. Consequently, there may not be enough data to determine a trend or tendency in the patient's changing visual field. The present system addresses this issue by providing the synthesized/derived visual field tests to fill in such gaps. For example, although a patient might have skipped taking a visual field at a particular clinic visit (or particular month/time), the patient may have taken a retina image (e.g., OCT, OCTA, fundus image, FA, etc.) at that clinic visit (or within a predefined time frame, e.g., month or other set number of weeks/days). In this case, the taken retina image may be used to extract a derived visual field. This derived visual field may then be used in place of a true functional visual field in a VF-related analysis. For example, such derived visual fields may be used to create additional training sets (e.g., used as a VF target output VFTR*i* in a particular training pair TPi, as illustrated in FIG. 2) in additional training sessions of the neural network or to train another neural network. That is, derived visual fields may be used as training data (in place of, or in addition to, previously taken, true functional visual field results) in the training configurations of FIGS. 2 and/or 4.

**FIG. 5** provides an example of derived visual fields used in VF-related analysis. The example plot of FIG. 5 shows a patient's degrading visual field sensitivity over time, and illustrates how having a patient's visual field test history may be helpful in predicting (forecasting) the patient's present, or future, visual field (e.g., predict visual field sensitivity measures, such as based on threshold value(s) for a given test point). The vertical axis may correspond to measures of a patient's visual sensitivity, and the horizontal axis may correspond to the passage of time, such as a sequence of prescribed visual field test dates or scheduled clinic visits. In the present example, true prior VF test results are shown as solid dots, and synthesized (derived) VF results, such as based on biometric measures or other non-traditional functional visual field data, from the patient's previous clinic visits are shown as circles. A plot of Prior VF Test Sensitivity results vs Time helps to illustrate the patient's expected threshold at time "x". Such a prediction would not be possible if only true prior VF test results (solid dots) were used, which would indicate a linear progression, as illustrated by dash line Ln1, but the addition of synthesized VF results (circles) as additional "VF priors" to fill-in gaps in testing times reveals a more logarithmic, or curved, plot (indicated by dash curve Crv1) that better predicts future VF values for time "x". That is, the collection of derived and true visual fields may be input to a VF forecast system, which uses the inputs to predict a patient's current or future visual field. Such a VF forecast system may be embodied by a computing system implementing any number of forecasting techniques, such as machine learning (e.g., linear regression) and/or deep learning (e.g., recurrent neural networks).

**FIG. 6** illustrates a VF forecast system 21 in accord with the present invention. In the present example, VF priors are desired for 9 time slots/intervals TS1 to TS9, in order to better forecast a visual field VFTh_out for a subsequent time slot TS10. In the present example, true VF test results are available for time slots TS1, TS3, TS4, TS6, ST7, and ST8, but no true VF test results are available for time slots TS2, TS5 and TS9 due to gaps in the VF history record. Assuming that the patient has image data (biometric/physical measures) corresponding to the missing time slots (e.g. the patient took a retina image/scan, but did not take a VF test at the prescribed time slot), the present system may be used to synthesize VF priors for the missing time slots TS2, TS5 and TS9. The collection of true and synthesized VF priors may be submitted to forecast tool 21 (sequentially or in parallel), which may then output a predicted visual field VFTh_out for time slot TS10. Output VFTh_out may be submitted to block 13 as a derived visual field VFTh_out in FIG. 1.

A preliminary proof-of-concept study was conducted to evaluate the performance of using structure-derived visual field priors (S-priors) for simulated visual fields (VFs). Qualified (e.g., retrospective) data from 1399 subjects (single eyes) from a Singapore population study were used in this study. Data from the Humphrey Field Analyzer (HFA2i)^{®} (ZEISS, Dublin, CA) SITA Standard 24-2 VFs and the CIRRUS^{®} HD-OCT (ZEISS, Dublin, CA), including Optic cubes, were collected at the study visit. Seventy percent of the eyes were used to train regressors (e.g., a random forest regressor) to predict a 54-point VF. A random forest (RF) using the 256-point circumpapillary retinal nerve fiber layer data and age was constructed. A simplified mixed-scale dense convolutional neural network (CNN) using the RNFL thickness map was constructed, see for example, Pelt et al., "A Mixed-Scale Dense Convolutional Neural Network for Image Analysis," PNAS, 2018, 115 (2), 254-259, herein incorporated in its entirety by reference. The remaining 30% of the eyes were used to predict S-priors and to provide input fields to a VF simulator.

The VF simulator implemented a Bayesian ZEST using a bi-modal starting probability distribution (SPD) with no prior (*ZEST*)*,* as described in "Targeted Spatial Sampling Using GOANNA Improves Detection of Visual Field Progression," (Chong et al., Ophthalmic Physiol Opt, 2015, Mar;35(2):155-69), except the normal mode was instead centered on age normal values determined from a normal cohort of 118 eyes, as described in "Exploring the Structure-Function Relationship for Perimetry Stimulus Sizes III, V and VI and OCT in Early Glaucoma," Flanagan et al., ARVO (Association for Research in Vision and Ophthalmology) Abstract, Investigative Ophthalmology & Visual Science (IOVS), Sept 2016, Volume 57, 376, herein incorporate in its entirety by reference.

*ZESTs* using a uni-modal SPD designed for custom priors centered on both types of S-priors were also simulated (e.g., *ZEST-RF, ZEST-CNN*)*.* Slopes of frequency of seeing responses were modeled, as described "Response Variability in the Visual Field: Comparison of Optic Neuritis, Glaucoma, Ocular Hypertension, and Normal Eyes," (Henson et al., IOVS, Feb 2000, Vol. 41, 417-421), herein incorporate in its entirety by reference. False answer rates were set to 0%, 5%, and 20% as 3 types of responders. Performance between simulated (e.g., synthesized) and true VFs was evaluated by observing the mean absolute error (MAE) between simulated and true VFs and the total number of questions. The two locations nearest the blind spot were excluded from the analyses. Significance testing (2 one-sided, paired t-tests, α = 0.05) for inter-strategy equivalence versus *ZEST* was performed using limits of agreement of ±5% dB for MAE and ±5% for total questions.

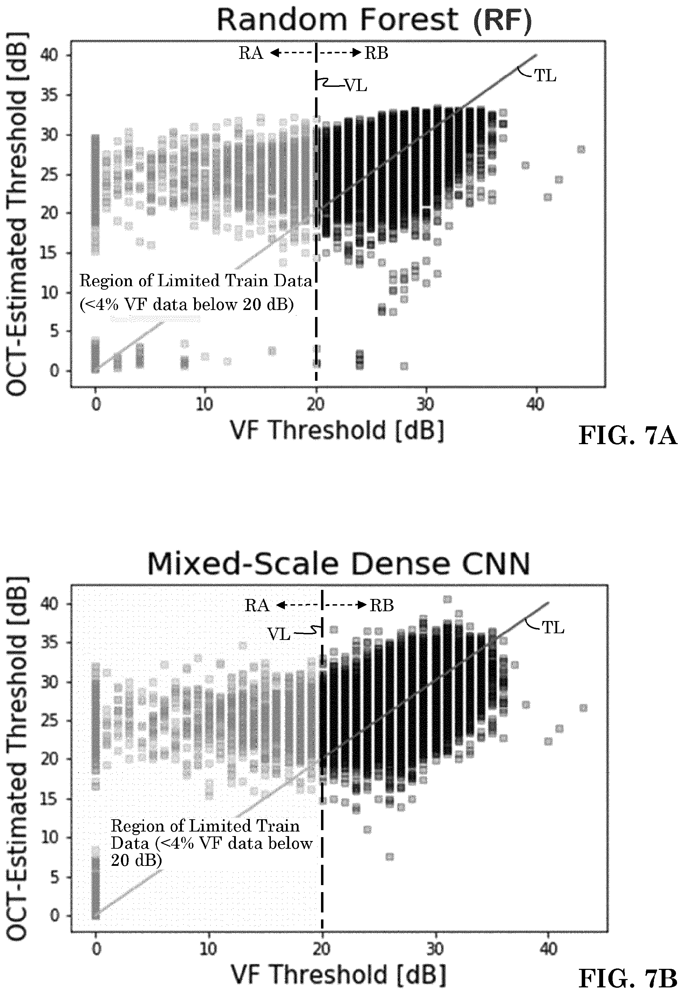

The results show that Mean VF MD were -1.8±2.4 dB and -2.7±2.7 dB for training and test sets, respectively (p<0.001). **FIGs. 7A and 7B** are plots of (derived) OCT-Estimated Threshold vs (true) VF Threshold for example applications using Random Forest (FIG. 7A) and Neural Network (FIG. 7B) machine learning models. Because this is a proof of concept application, availability of training data was limited, particularly for certain threshold values. In each plot, dotted vertical line VL provides visual indicator separating a region of little training data RA (e.g., at lower thresholds) from a region RB where more training data was available (e.g., at more normal thresholds). As it would be understood, target line TL indicates a desired distribution/trend to indicate an equivalence between true and derived thresholds. Both FIGS. 7A and 7B show that the present simple models performed better (e.g., the plotted data distribution follows target line TL better) in region RB where more training data was available than in region RA (e.g., the present simple models appear to perform better at more normal thresholds than at lower thresholds). It is likely that providing additional training data, particularly at lower thresholds, would improve the present models and render better results. Irrespective, FIG. 7B suggests that the (deep learning) neural network (CNN) model may achieve better results (e.g., plotted data better follow target line TL) than the random forest (RF) model.

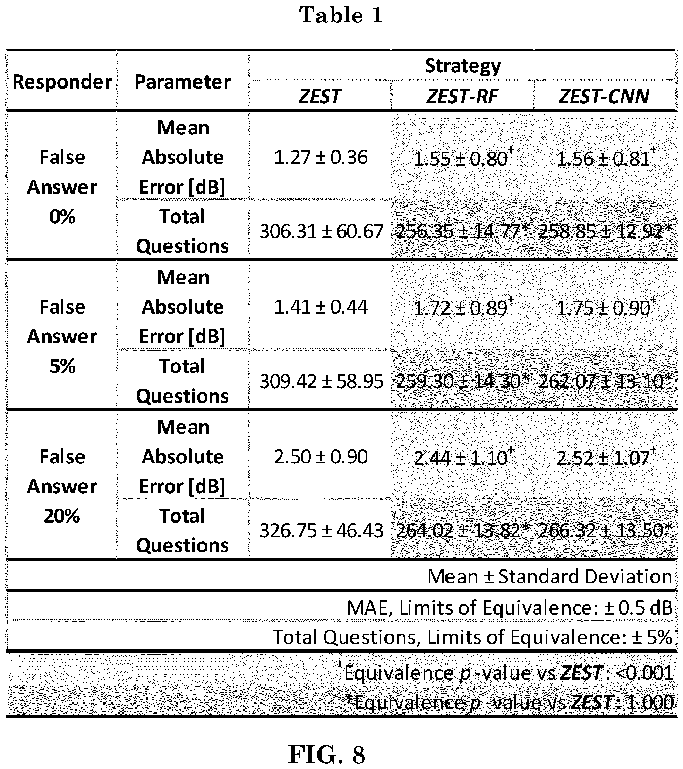

However, **FIG. 8** illustrates a Table 1 indicating that overall MAEs for *ZEST-RF* and *ZEST-CNN* were statistically equivalent to *ZEST* (p<0.001). Total questions were reduced by 16-19% for *ZEST-CNN* vs*. ZEST.* These findings suggest that even a simple model with limited/unbalanced data that predicts VFs from biometric/structural data (e.g., OCT data and/or fundus images) can reduce the duration of the initial VF exam in this population with comparable error. With more data representing a clinical population and more refined models, performance may be further improved.

Hereinafter is provided a description of various hardware and architectures suitable for the present invention.

### Visual Field Test System

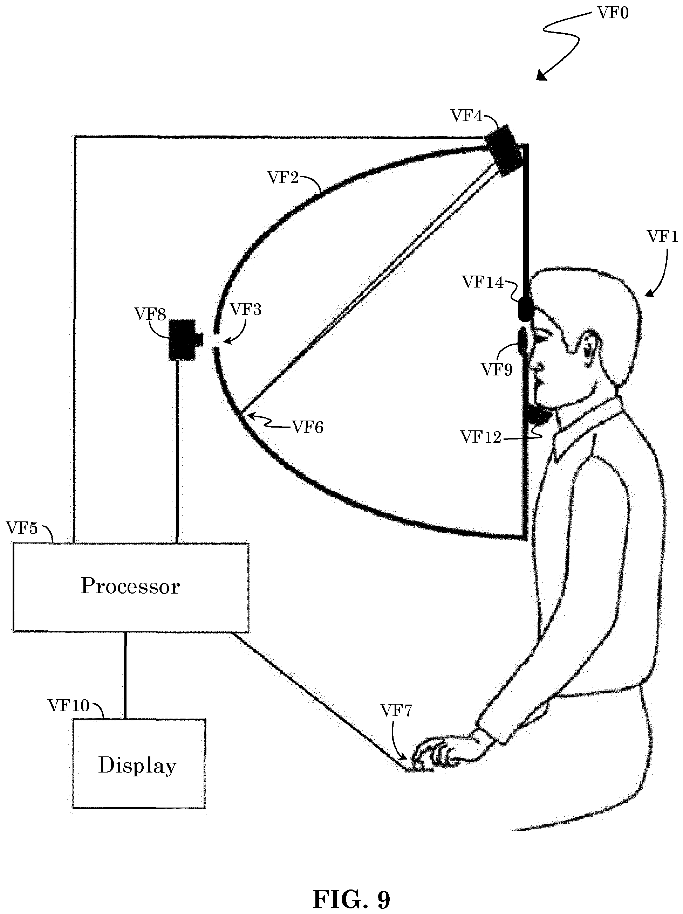

The improvements described herein may be used in conjunction with any type of visual field tester/system, e.g., perimeter. One such system is a "bowl" visual field tester VF0, as illustrated in **FIG. 9****.** A subject (e.g., patient) VF1 is shown observing a hemispherical projection screen (or other type of display) VF2 generally shaped as a bowl, for which the tester VF0 is so termed. Typically, the subject is instructed to fixate at a point at the center of the hemispherical screen VF3. The subject rests his/her head on a patient support, which may include a chin rest VF12 and/or a forehead rest VF14. For instance, the subject rests his/her head on the chin rest VF12 and places his/her forehead against the forehead rest VF14. Optionally, the chin rest VF12 and the forehead rest VF14 may be moved together or independently of one another to correctly fixate/position the patient's eye, e.g., relative to a trial lens holder VF9 that may hold a lens through which the subject may view screen VF2. For example, the chin rest and headrest may move independently in the vertical direction to accommodate different patient head sizes and move together in the horizontal and/or vertical direction to correctly position the head. However, this is not limiting, and other arrangements/movements can be envisioned by one skilled in the art.

A projector, or other imaging device, VF4 under control of a processor VF5 displays a series of test stimuli (e.g., test points of any shape) VF6 onto the screen VF2. The subject VF1 indicates that he/she sees a stimulus VF6 by actuating a user input VF7 (e.g., depressing an input button). This subject response may be recorded by processor VF5, which may function to evaluate the visual field of an eye based on the subject's responses, e.g., determine the size, position, and/or intensity of a test stimulus VF6 at which it can no longer be seen by the subject VF1, and thereby determine the (visible) threshold of the test stimulus VF6. A camera VF8 may be used to capture the gaze (e.g., gaze direction) of the patient throughout the test. Gaze direction may be used for patient alignment and/or to ascertain the patient's adherence to proper test procedures. In the present example, the camera VF8 is located on the Z-axis relative to the patient's eye (e.g. relative to trial lens holder VF9) and behind the bowl (of screen VF2) for capturing live images(s) or video of the patient's eye. In other embodiments, this camera may be located off this Z-axis. The images from the gaze camera VF8 can optionally be displayed on a second display VF10 to a clinician (who may also be interchangeably referred to herein as a technician) for aid in patient alignment or test verification. The camera VF8 may record and store one or more images of the eye during each stimulus presentation. This may lead to a collection of anywhere from tens to hundreds of images per visual field test, depending on the testing conditions. Alternatively, the camera VF8 may record and store a full length movie during the test and provide time stamps indicating when each stimulus is presented. Additionally, images may also be collected between stimulus presentations to provide details on the subject's overall attention throughout the VF test's duration.

Trial lens holder VF9 may be positioned in front of the patient's eye to correct for any refractive error in the eye. Optionally, the lens holder VF9 may carry or hold a liquid trial lens (see for example US Patent No. 8,668,338, the contents of which are hereby incorporated in their entirety by reference), which may be utilized to provide variable refractive correction for the patient VF1. However, it should be noted that the present invention is not limited to using a liquid trial lens for refraction correction and other conventional/standard trial lenses known in the art may also be used.

In some embodiments, one or more light sources (not shown) may be positioned in front of the eye of the subject VF1, which create reflections from ocular surfaces such as the cornea. In one variation, the light sources may be light-emitting diodes (LEDs).

While FIG. 9 shows a projection type visual field tester VF0, the invention described herein may be used with other types of devices (visual field testers), including those that generate images through a liquid crystal display (LCD) or other electronic display (see for example U.S. Patent No. 8,132,916. Other types of visual field testers include, for example, flat-screen testers, miniaturized testers, and binocular visual field testers. Examples of these types of testers may be found in US Pat. 8,371,696, US Pat. 5,912,723, US Pat. No. 8,931,905, US designed patent D472637.

Visual field tester VF0 may incorporate an instrument-control system (e.g. running an algorithm, which may be software, code, and/or routine) that uses hardware signals and a motorized positioning system to automatically position the patient's eye at a desired position, e.g., the center of a refraction correction lens at lens holder VF9. For example, stepper motors may move chin rest VF12 and the forehead rest VF14 under software control. A rocker switch may be provided to enable the attending technician to adjust the patient's head position by causing the chin rest and forehead stepper motors to operate. A manually moveable refraction lens may also be placed in front of the patient's eye on lens holder VF9 as close to the patient's eye as possible without adversely affecting the patient's comfort. Optionally, the instrument control algorithm may pause perimetry test execution while chin rest and/or forehead motor movements are under way if such movements would disrupt test execution.

### Fundus Imaging System

Two categories of imaging systems used to image the fundus are flood illumination imaging systems (or flood illumination imagers) and scan illumination imaging systems (or scan imagers). Flood illumination imagers flood with light an entire field of view (FOV) of interest of a specimen at the same time, such as by use of a flash lamp, and capture a full-frame image of the specimen (e.g., the fundus) with a full-frame camera (e.g., a camera having a two-dimensional (2D) photo sensor array of sufficient size to capture the desired FOV, as a whole). For example, a flood illumination fundus imager would flood the fundus of an eye with light, and capture a full-frame image of the fundus in a single image capture sequence of the camera. A scan imager provides a scan beam that is scanned across a subject, e.g., an eye, and the scan beam is imaged at different scan positions as it is scanned across the subject creating a series of image-segments that may be reconstructed, e.g., montaged, to create a composite image of the desired FOV. The scan beam could be a point, a line, or a two-dimensional area such a slit or broad line.

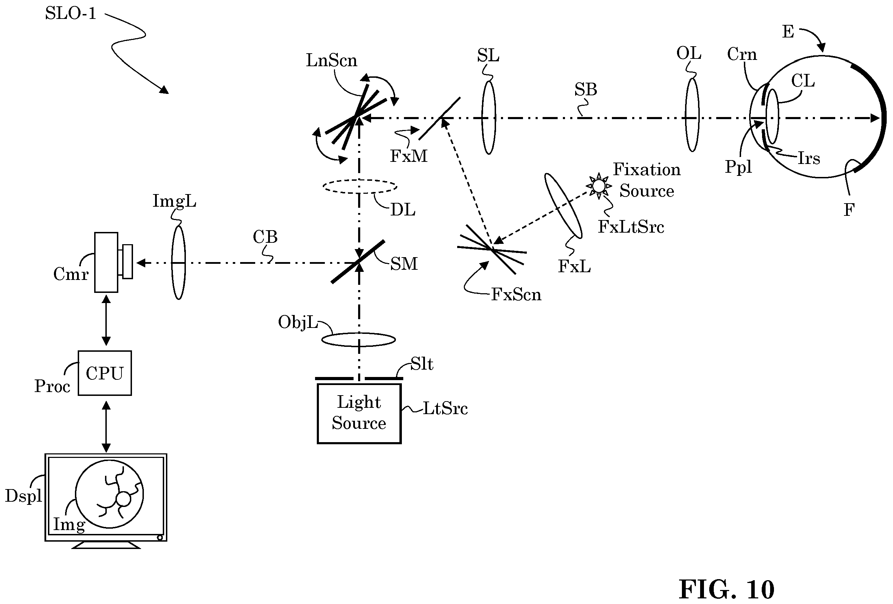

**FIG. 10** illustrates an example of a slit scanning ophthalmic system SLO-1 for imaging a fundus F, which is the interior surface of an eye E opposite the eye lens (or crystalline lens) CL and may include the retina, optic disc, macula, fovea, and posterior pole. In the present example, the imaging system is in a so-called "scan-descan" configuration, wherein a scanning line beam SB traverses the optical components of the eye E (including the cornea Crn, iris Irs, pupil Ppl, and crystalline lens CL) to be scanned across the fundus F. In the case of a flood fundus imager, no scanner is needed, and the light is applied across the entire, desired field of view (FOV) at once. Other scanning configurations are known in the art, and the specific scanning configuration is not critical to the present invention. As depicted, the imaging system includes one or more light sources LtSrc, preferably a multi-color LED system or a laser system in which the etendue has been suitably adjusted. An optional slit Slt (adjustable or static) is positioned in front of the light source LtSrc and may be used to adjust the width of the scanning line beam SB. Additionally, slit Slt may remain static during imaging or may be adjusted to different widths to allow for different confocality levels and different applications either for a particular scan or during the scan for use in suppressing reflexes. An optional objective lens ObjL may be placed in front of the slit Slt. The objective lens ObjL can be any one of state-of-the-art lenses including but not limited to refractive, diffractive, reflective, or hybrid lenses/systems. The light from slit Slt passes through a pupil splitting mirror SM and is directed towards a scanner LnScn. It is desirable to bring the scanning plane and the pupil plane as near together as possible to reduce vignetting in the system. Optional optics DL may be included to manipulate the optical distance between the images of the two components. Pupil splitting mirror SM may pass an illumination beam from light source LtSrc to scanner LnScn, and reflect a detection beam from scanner LnScn (e.g., reflected light returning from eye E) toward a camera Cmr. A task of the pupil splitting mirror SM is to split the illumination and detection beams and to aid in the suppression of system reflexes. The scanner LnScn could be a rotating galvo scanner or other types of scanners (e.g., piezo or voice coil, micro-electromechanical system (MEMS) scanners, electro-optical deflectors, and/or rotating polygon scanners). Depending on whether the pupil splitting is done before or after the scanner LnScn, the scanning could be broken into two steps wherein one scanner is in an illumination path and a separate scanner is in a detection path. Specific pupil splitting arrangements are described in detail in US Patent No. 9,456,746, which is herein incorporated in its entirety by reference.

From the scanner LnScn, the illumination beam passes through one or more optics, in this case a scanning lens SL and an ophthalmic or ocular lens OL, that allow for the pupil of the eye E to be imaged to an image pupil of the system. Generally, the scan lens SL receives a scanning illumination beam from the scanner LnScn at any of multiple scan angles (incident angles), and produces scanning line beam SB with a substantially flat surface focal plane (e.g., a collimated light path). Ophthalmic lens OL may focus the scanning line beam SB onto the fundus F (or retina) of eye E and image the fundus. In this manner, scanning line beam SB creates a traversing scan line that travels across the fundus F. One possible configuration for these optics is a Kepler type telescope wherein the distance between the two lenses is selected to create an approximately telecentric intermediate fundus image (4-f configuration). The ophthalmic lens OL could be a single lens, an achromatic lens, or an arrangement of different lenses. All lenses could be refractive, diffractive, reflective or hybrid as known to one skilled in the art. The focal length(s) of the ophthalmic lens OL, scan lens SL and the size and/or form of the pupil splitting mirror SM and scanner LnScn could be different depending on the desired field of view (FOV), and so an arrangement in which multiple components can be switched in and out of the beam path, for example by using a flip in optic, a motorized wheel, or a detachable optical element, depending on the field of view can be envisioned. Since the field of view change results in a different beam size on the pupil, the pupil splitting can also be changed in conjunction with the change to the FOV. For example, a 45° to 60° field of view is a typical, or standard, FOV for fundus cameras. Higher fields of view, e.g., a widefield FOV, of 60°-120°, or more, may also be feasible. A widefield FOV may be desired for a combination of the Broad-Line Fundus Imager (BLFI) with another imaging modalities such as optical coherence tomography (OCT). The upper limit for the field of view may be determined by the accessible working distance in combination with the physiological conditions around the human eye. Because a typical human retina has a FOV of 140° horizontal and 80°-100° vertical, it may be desirable to have an asymmetrical field of view for the highest possible FOV on the system.

The scanning line beam SB passes through the pupil Ppl of the eye E and is directed towards the retinal, or fundus, surface F. The scanner LnScn1 adjusts the location of the light on the retina, or fundus, F such that a range of transverse locations on the eye E are illuminated. Reflected or scattered light (or emitted light in the case of fluorescence imaging) is directed back along as similar path as the illumination to define a collection beam CB on a detection path to camera Cmr.

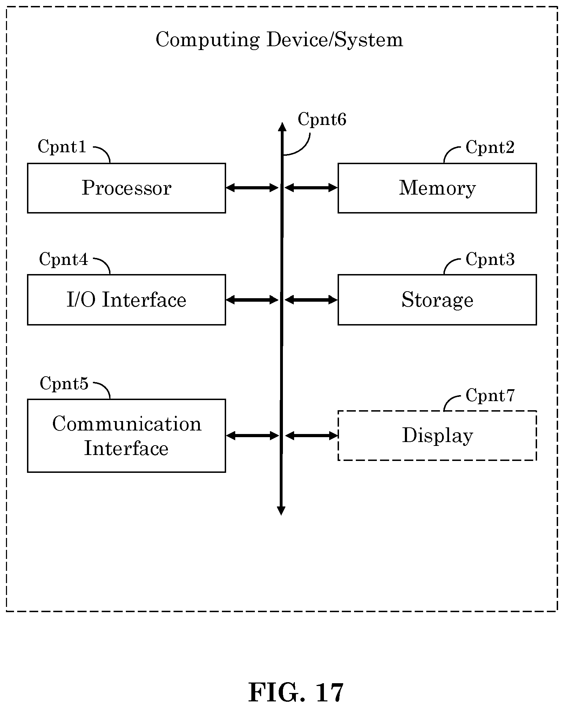

In the "scan-descan" configuration of the present, exemplary slit scanning ophthalmic system SLO-1, light returning from the eye E is "descanned" by scanner LnScn on its way to pupil splitting mirror SM. That is, scanner LnScn scans the illumination beam from pupil splitting mirror SM to define the scanning illumination beam SB across eye E, but since scanner LnScn also receives returning light from eye E at the same scan position, scanner LnScn has the effect of descanning the returning light (e.g., cancelling the scanning action) to define a non-scanning (e.g., steady or stationary) collection beam from scanner LnScn to pupil splitting mirror SM, which folds the collection beam toward camera Cmr. At the pupil splitting mirror SM, the reflected light (or emitted light in the case of fluorescence imaging) is separated from the illumination light onto the detection path directed towards camera Cmr, which may be a digital camera having a photo sensor to capture an image. An imaging (e.g., objective) lens ImgL may be positioned in the detection path to image the fundus to the camera Cmr. As is the case for objective lens ObjL, imaging lens ImgL may be any type of lens known in the art (e.g., refractive, diffractive, reflective or hybrid lens). Additional operational details, in particular, ways to reduce artifacts in images, are described in PCT Publication No. WO2016/124644. The camera Cmr captures the received image, e.g., it creates an image file, which can be further processed by one or more (electronic) processors or computing devices (e.g., the computer system shown in FIG. 17). Thus, the collection beam (returning from all scan positions of the scanning line beam SB) is collected by the camera Cmr, and a full-frame image Img may be constructed from a composite of the individually captured collection beams, such as by montaging. However, other scanning configuration are also contemplated, including ones where the illumination beam is scanned across the eye E and the collection beam is scanned across a photo sensor array of the camera. PCT Publication WO 2012/059236 and US Patent Publication No. 2015/0131050, herein incorporated by reference, describe several embodiments of slit scanning ophthalmoscopes including various designs where the returning light is swept across the camera's photo sensor array and where the returning light is not swept across the camera's photo sensor array.

In the present example, the camera Cmr is connected to a processor (e.g., processing module) Proc and a display (e.g., displaying module, computer screen, electronic screen, etc.) Dspl, both of which can be part of the image system itself, or may be part of separate, dedicated processing and/or displaying unit(s), such as a computer system wherein data is passed from the camera Cmr to the computer system over a cable or computer network including wireless networks. The display and processor can be an all in one unit. The display can be a traditional electronic display/screen or of the touch screen type and can include a user interface for displaying information to and receiving information from an instrument operator, or user. The user can interact with the display using any type of user input device as known in the art including, but not limited to, mouse, knobs, buttons, pointer, and touch screen.

It may be desirable for a patient's gaze to remain fixed while imaging is carried out. One way to achieve this is to provide a fixation target that the patient can be directed to stare at. Fixation targets can be internal or external to the instrument depending on what area of the eye is to be imaged. One embodiment of an internal fixation target is shown in FIG. 10. In addition to the primary light source LtSrc used for imaging, a second optional light source FxLtSrc, such as one or more LEDs, can be positioned such that a light pattern is imaged to the retina using lens FxL, scanning element FxScn and reflector/mirror FxM. Fixation scanner FxScn can move the position of the light pattern and reflector FxM directs the light pattern from fixation scanner FxScn to the fundus F of eye E. Preferably, fixation scanner FxScn is position such that it is located at the pupil plane of the system so that the light pattern on the retina/fundus can be moved depending on the desired fixation location.

Slit-scanning ophthalmoscope systems are capable of operating in different imaging modes depending on the light source and wavelength selective filtering elements employed. True color reflectance imaging (imaging similar to that observed by the clinician when examining the eye using a hand-held or slit lamp ophthalmoscope) can be achieved when imaging the eye with a sequence of colored LEDs (red, blue, and green). Images of each color can be built up in steps with each LED turned on at each scanning position or each color image can be taken in its entirety separately. The three, color images can be combined to display the true color image, or they can be displayed individually to highlight different features of the retina. The red channel best highlights the choroid, the green channel highlights the retina, and the blue channel highlights the anterior retinal layers. Additionally, light at specific frequencies (e.g., individual colored LEDs or lasers) can be used to excite different fluorophores in the eye (e.g., autofluorescence) and the resulting fluorescence can be detected by filtering out the excitation wavelength.

The fundus imaging system can also provide an infrared reflectance image, such as by using an infrared laser (or other infrared light source). The infrared (IR) mode is advantageous in that the eye is not sensitive to the IR wavelengths. This may permit a user to continuously take images without disturbing the eye (e.g., in a preview/alignment mode) to aid the user during alignment of the instrument. Also, the IR wavelengths have increased penetration through tissue and may provide improved visualization of choroidal structures. In addition, fluorescein angiography (FA) and indocyanine green (ICG) angiography imaging can be accomplished by collecting images after a fluorescent dye has been injected into the subject's bloodstream. For example, in FA (and/or ICG) a series of time-lapse images may be captured after injecting a light-reactive dye (e.g., fluorescent dye) into a subject's bloodstream. It is noted that care must be taken since the fluorescent dye may lead to a life-threatening allergic reaction in a portion of the population. High contrast, greyscale images are captured using specific light frequencies selected to excite the dye. As the dye flows through the eye, various portions of the eye are made to glow brightly (e.g., fluoresce), making it possible to discern the progress of the dye, and hence the blood flow, through the eye.

### Optical Coherence Tomography Imaging System

In addition to fundus photography, fundus auto-fluorescence (FAF), fluorescein angiography (FA), ophthalmic images may also be created by other imaging modalities, such as, optical coherence tomography (OCT), OCT angiography (OCTA), and/or ocular ultrasonography. The present invention, or at least portions of the present invention with minor modification(s) as it would be understood in the art, may be applied to these other ophthalmic imaging modalities. More specifically, the present invention may also be applied to ophthalmic images produces by an OCT/OCTA system producing OCT and/or OCTA images. For instance, the present invention may be applied to *en face* OCT/OCTA images. Examples of fundus imagers are provided in US Pats. 8,967,806 and 8,998,411, examples of OCT systems are provided in U.S. Pats. 6,741,359 and 9,706,915, and examples of an OCTA imaging system may be found in U.S. Pats. 9,700,206 and 9,759,544, all of which are herein incorporated in their entirety by reference. For the sake of completeness, an exemplary OCT/OCTA system is provided herein.

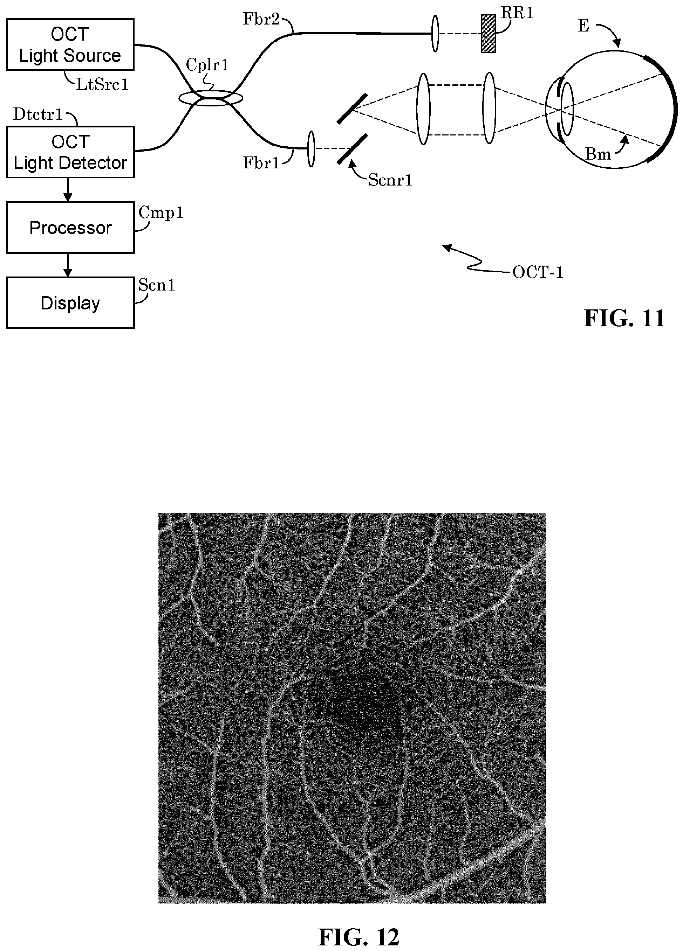

**FIG. 11** illustrates a generalized frequency domain optical coherence tomography (FD-OCT) system used to collect 3-D image data of the eye suitable for use with the present invention. An FD-OCT system OCT_1 includes a light source, LtSrc1. Typical light sources include, but are not limited to, broadband light sources with short temporal coherence lengths or swept laser sources. A beam of light from light source LtSrc 1 is routed, typically by optical fiber Fbr1, to illuminate a sample, e.g., eye E; a typical sample being tissues in the human eye. The light source LrSrc1 can be either a broadband light source with short temporal coherence length in the case of spectral domain OCT (SD-OCT) or a wavelength tunable laser source in the case of swept source OCT (SS-OCT). The light may be scanned, typically with a scanner Scnr1 between the output of the optical fiber Fbr1 and the sample E, so that the beam of light (dashed line Bm) is scanned laterally (in x and y) over the region of the sample to be imaged. In the case of a full-field OCT, no scanner is needed and the light is applied across the entire, desired field of view (FOV) at once. Light scattered from the sample is collected, typically into the same optical fiber Fbr1 used to route the light for illumination. Reference light derived from the same light source LtSrc1 travels a separate path, in this case involving optical fiber Fbr2 and retro-reflector RR1 with an adjustable optical delay. Those skilled in the art will recognize that a transmissive reference path can also be used and that the adjustable delay could be placed in the sample or reference arm of the interferometer. Collected sample light is combined with reference light, typically in a fiber coupler Cplr1, to form light interference in an OCT light detector Dtctr1 (e.g., photodetector array, digital camera, etc.). Although a single fiber port is shown going to the detector Dtctr1, those skilled in the art will recognize that various designs of interferometers can be used for balanced or unbalanced detection of the interference signal. The output from the detector Dtctr1is supplied to a processor Cmp1 (e.g., computing device) that converts the observed interference into depth information of the sample. The depth information may be stored in a memory associated with the processor Cmp1 and/or displayed on a display (e.g., computer/electronic display/screen) Scn1. The processing and storing functions may be localized within the OCT instrument or functions may be performed on an external processing unit (e.g., the computer system shown in FIG. 17) to which the collected data is transferred. This unit could be dedicated to data processing or perform other tasks which are quite general and not dedicated to the OCT device. The processor Cmp1 may contain, for example, a field-programmable gate array (FPGA), a digital signal processor (DSP), an application specific integrated circuit (ASIC), a graphics processing unit (GPU), a system on chip (SoC), a central processing unit (CPU), a general purpose graphics processing unit (GPGPU), or a combination thereof, that performs some, or the entire data processing steps, prior to passing on to the host processor or in a parallelized fashion.

The sample and reference arms in the interferometer could consist of bulk-optics, fiber-optics, or hybrid bulk-optic systems and could have different architectures such as Michelson, Mach-Zehnder or common-path based designs as would be known by those skilled in the art. Light beam as used herein should be interpreted as any carefully directed light path. Instead of mechanically scanning the beam, a field of light can illuminate a one or two-dimensional area of the retina to generate the OCT data (see for example, U.S. Patent 9332902; D. Hillmann et al, "Holoscopy - Holographic Optical Coherence Tomography," Optics Letters, 36(13): 2390 2011; Y. Nakamura, et al, "High-Speed Three Dimensional Human Retinal Imaging by Line Field Spectral Domain Optical Coherence Tomography," Optics Express, 15(12):7103 2007; Blazkiewicz et al, "Signal-To-Noise Ratio Study of Full-Field Fourier-Domain Optical Coherence Tomography," Applied Optics, 44(36):7722 (2005)). In time-domain systems, the reference arm needs to have a tunable optical delay to generate interference. Balanced detection systems are typically used in TD-OCT and SS-OCT systems, while spectrometers are used at the detection port for SD-OCT systems. The invention described herein could be applied to any type of OCT system. Various aspects of the invention could apply to any type of OCT system or other types of ophthalmic diagnostic systems and/or multiple ophthalmic diagnostic systems including but not limited to fundus imaging systems, visual field test devices, and scanning laser polarimeters.

In Fourier Domain optical coherence tomography (FD-OCT), each measurement is the real-valued spectral interferogram (Sj(k)). The real-valued spectral data typically goes through several post-processing steps including background subtraction, dispersion correction, etc. The Fourier transform of the processed interferogram, results in a complex valued OCT signal output Aj(z)=|Aj|eiφ. The absolute value of this complex OCT signal, |Aj|, reveals the profile of scattering intensities at different path lengths, and therefore scattering as a function of depth (z-direction) in the sample. Similarly, the phase, φj can also be extracted from the complex valued OCT signal. The profile of scattering as a function of depth is called an axial scan (A-scan). A set of A-scans measured at neighboring locations in the sample produces a cross-sectional image (tomogram or B-scan) of the sample. A collection of B-scans collected at different transverse locations on the sample makes up a data volume or cube. For a particular volume of data, the term fast axis refers to the scan direction along a single B-scan whereas slow axis refers to the axis along which multiple B-scans are collected. The term "cluster scan" may refer to a single unit or block of data generated by repeated acquisitions at the same (or substantially the same) location (or region) for the purposes of analyzing motion contrast, which may be used to identify blood flow. A cluster scan can consist of multiple A-scans or B-scans collected with relatively short time separations at approximately the same location(s) on the sample. Since the scans in a cluster scan are of the same region, static structures remain relatively unchanged from scan to scan within the cluster scan, whereas motion contrast between the scans that meets predefined criteria may be identified as blood flow. A variety of ways to create B-scans are known in the art including but not limited to: along the horizontal or x-direction, along the vertical or y-direction, along the diagonal of x and y, or in a circular or spiral pattern. B-scans may be in the x-z dimensions but may be any cross-sectional image that includes the z-dimension.

In OCT Angiography, or Functional OCT, analysis algorithms may be applied to OCT data collected at the same, or approximately the same, sample locations on a sample at different times (e.g., a cluster scan) to analyze motion or flow (see for example US Patent Publication Nos. 2005/0171438, 2012/0307014, 2010/0027857, 2012/0277579 and US Patent No. 6,549,801, all of which are herein incorporated in their entirety by reference). An OCT system may use any one of a number of OCT angiography processing algorithms (e.g., motion contrast algorithms) to identify blood flow. For example, motion contrast algorithms can be applied to the intensity information derived from the image data (intensity-based algorithm), the phase information from the image data (phase-based algorithm), or the complex image data (complex-based algorithm). An *en face* image is a 2D projection of 3D OCT data (e.g., by averaging the intensity of each individual A-scan, such that each A-scan defines a pixel in the 2D projection). Similarly, an *en face* vasculature image is an image displaying motion contrast signal in which the data dimension corresponding to depth (e.g., z-direction along an A-scan) is displayed as a single representative value (e.g., a pixel in a 2D projection image), typically by summing or integrating all or an isolated portion of the data (see for example US Patent No. 7,301,644 herein incorporated in its entirety by reference). OCT systems that provide an angiography imaging functionality may be termed OCT angiography (OCTA) systems.

**FIG. 12** shows an example of an *en face* vasculature image. After processing the data to highlight motion contrast using any of the motion contrast techniques known in the art, a range of pixels corresponding to a given tissue depth from the surface of internal limiting membrane (ILM) in retina, may be summed to generate the *en face* (e.g., frontal view) image of the vasculature.

### Neural Networks

As discussed above, the present invention may use a neural network (NN) machine learning (ML) model. For the sake of completeness, a general discussion of neural networks is provided herein. The present invention may use any, singularly or in combination, of the below described neural network architecture(s). A neural network, or neural net, is a (nodal) network of interconnected neurons, where each neuron represents a node in the network. Groups of neurons may be arranged in layers, with the outputs of one layer feeding forward to a next layer in a multilayer perceptron (MLP) arrangement. MLP may be understood to be a feedforward neural network model that maps a set of input data onto a set of output data.

**FIG. 13** illustrates an example of a multilayer perceptron (MLP) neural network. Its structure may include multiple hidden (e.g., internal) layers HL1 to HLn that map an input layer InL (that receives a set of inputs (or vector input) in_1 to in_3) to an output layer OutL that produces a set of outputs (or vector output), e.g., out_1 and out_2. Each layer may have any given number of nodes, which are herein illustratively shown as circles within each layer. In the present example, the first hidden layer HL1 has two nodes, while hidden layers HL2, HL3, and HLn each have three nodes. Generally, the deeper the MLP (e.g., the greater the number of hidden layers in the MLP), the greater its capacity to learn. The input layer InL receives a vector input (illustratively shown as a three-dimensional vector consisting of in_1, in_2 and in_3), and may apply the received vector input to the first hidden layer HL1 in the sequence of hidden layers. An output layer OutL receives the output from the last hidden layer, e.g., HLn, in the multilayer model, processes its inputs, and produces a vector output result (illustratively shown as a two-dimensional vector consisting of out_1 and out_2).

Typically, each neuron (or node) produces a single output that is fed forward to neurons in the layer immediately following it. But each neuron in a hidden layer may receive multiple inputs, either from the input layer or from the outputs of neurons in an immediately preceding hidden layer. In general, each node may apply a function to its inputs to produce an output for that node. Nodes in hidden layers (e.g., learning layers) may apply the same function to their respective input(s) to produce their respective output(s). Some nodes, however, such as the nodes in the input layer InL receive only one input and may be passive, meaning that they simply relay the values of their single input to their output(s), e.g., they provide a copy of their input to their output(s), as illustratively shown by dotted arrows within the nodes of input layer InL.

For illustration purposes, **FIG**. **14** shows a simplified neural network consisting of an input layer InL', a hidden layer HL 1', and an output layer OutL'. Input layer InL' is shown having two input nodes i1 and i2 that respectively receive inputs Input_1 and Input_2 (e.g. the input nodes of layer InL' receive an input vector of two dimensions). The input layer InL' feeds forward to one hidden layer HL1' having two nodes h1 and h2, which in turn feeds forward to an output layer OutL' of two nodes o1 and o2. Interconnections, or links, between neurons (illustrative shown as solid arrows) have weights w1 to w8. Typically, except for the input layer, a node (neuron) may receive as input the outputs of nodes in its immediately preceding layer. Each node may calculate its output by multiplying each of its inputs by each input's corresponding interconnection weight, summing the products of it inputs, adding (or multiplying by) a constant defined by another weight or bias that may be associated with that particular node (e.g., node weights w9, w10, w11, w12 respectively corresponding to nodes h1, h2, o1, and o2), and then applying a non-linear function or logarithmic function to the result. The non-linear function may be termed an activation function or transfer function. Multiple activation functions are known the art, and selection of a specific activation function is not critical to the present discussion. It is noted, however, that operation of the ML model, or behavior of the neural net, is dependent upon weight values, which may be learned so that the neural network provides a desired output for a given input.

The neural net learns (e.g., is trained to determine) appropriate weight values to achieve a desired output for a given input during a training, or learning, stage. Before the neural net is trained, each weight may be individually assigned an initial (e.g., random and optionally non-zero) value, e.g. a random-number seed. Various methods of assigning initial weights are known in the art. The weights are then trained (optimized) so that for a given training vector input, the neural network produces an output close to a desired (predetermined) training vector output. For example, the weights may be incrementally adjusted in thousands of iterative cycles by a technique termed back-propagation. In each cycle of back-propagation, a training input (e.g., vector input or training input image/sample) is fed forward through the neural network to determine its actual output (e.g., vector output). An error for each output neuron, or output node, is then calculated based on the actual neuron output and a target training output for that neuron (e.g., a training output image/sample corresponding to the present training input image/sample). One then propagates back through the neural network (in a direction from the output layer back to the input layer) updating the weights based on how much effect each weight has on the overall error so that the output of the neural network moves closer to the desired training output. This cycle is then repeated until the actual output of the neural network is within an acceptable error range of the desired training output for the given training input. As it would be understood, each training input may require many back-propagation iterations before achieving a desired error range. Typically, an epoch refers to one back-propagation iteration (e.g., one forward pass and one backward pass) of all the training samples, such that training a neural network may require many epochs. Generally, the larger the training set, the better the performance of the trained ML model, so various data augmentation methods may be used to increase the size of the training set. For example, when the training set includes pairs of corresponding training input images and training output images, the training images may be divided into multiple corresponding image segments (or patches). Corresponding patches from a training input image and training output image may be paired to define multiple training patch pairs from one input/output image pair, which enlarges the training set. Training on large training sets, however, places high demands on computing resources, e.g. memory and data processing resources. Computing demands may be reduced by dividing a large training set into multiple mini-batches, where the mini-batch size defines the number of training samples in one forward/backward pass. In this case, and one epoch may include multiple mini-batches. Another issue is the possibility of a NN overfitting a training set such that its capacity to generalize from a specific input to a different input is reduced. Issues of overfitting may be mitigated by creating an ensemble of neural networks or by randomly dropping out nodes within a neural network during training, which effectively removes the dropped nodes from the neural network. Various dropout regulation methods, such as inverse dropout, are known in the art.

It is noted that the operation of a trained NN machine model is not a straightforward algorithm of operational/analyzing steps. Indeed, when a trained NN machine model receives an input, the input is not analyzed in the traditional sense. Rather, irrespective of the subject or nature of the input (e.g., a vector defining a live image/scan or a vector defining some other entity, such as a demographic description or a record of activity) the input will be subjected to the same predefined architectural construct of the trained neural network (e.g., the same nodal/layer arrangement, trained weight and bias values, predefined convolution/deconvolution operations, activation functions, pooling operations, etc.), and it may not be clear how the trained network's architectural construct produces its output. Furthermore, the values of the trained weights and biases are not deterministic and depend upon many factors, such as the amount of time the neural network is given for training (e.g., the number of epochs in training), the random starting values of the weights before training starts, the computer architecture of the machine on which the NN is trained, selection of training samples, distribution of the training samples among multiple mini-batches, choice of activation function(s), choice of error function(s) that modify the weights, and even if training is interrupted on one machine (e.g., having a first computer architecture) and completed on another machine (e.g., having a different computer architecture). The point is that the reasons why a trained ML model reaches certain outputs is not clear, and much research is currently ongoing to attempt to determine the factors on which a ML model bases its outputs. Therefore, the processing of a neural network on live data cannot be reduced to a simple algorithm of steps. Rather, its operation is dependent upon its training architecture, training sample sets, training sequence, and various circumstances in the training of the ML model.

In summary, construction of a NN machine learning model may include a learning (or training) stage and a classification (or operational) stage. In the learning stage, the neural network may be trained for a specific purpose and may be provided with a set of training examples, including training (sample) inputs and training (sample) outputs, and optionally including a set of validation examples to test the progress of the training. During this learning process, various weights associated with nodes and node-interconnections in the neural network are incrementally adjusted in order to reduce an error between an actual output of the neural network and the desired training output. In this manner, a multi-layer feedforward neural network (such as discussed above) may be made capable of approximating any measurable function to any desired degree of accuracy. The result of the learning stage is a (neural network) machine learning (ML) model that has been learned (e.g., trained). In the operational stage, a set of test inputs (or live inputs) may be submitted to the learned (trained) ML model, which may apply what it has learned to produce an output prediction based on the test inputs.

Like the regular neural networks of FIGS. 13 and 14, convolutional neural networks (CNN) are also made up of neurons that have learnable weights and biases. Each neuron receives inputs, performs an operation (e.g., dot product), and is optionally followed by a non-linearity. The CNN, however, may receive raw image pixels at one end (e.g., the input end) and provide classification (or class) scores at the other end (e.g., the output end). Because CNNs expect an image as input, they are optimized for working with volumes (e.g., pixel height and width of an image, plus the depth of the image, e.g., color depth such as an RGB depth defined of three colors: red, green, and blue). For example, the layers of a CNN may be optimized for neurons arranged in 3 dimensions. The neurons in a CNN layer may also be connected to a small region of the layer before it, instead of all of the neurons in a fully-connected NN. The final output layer of a CNN may reduce a full image into a single vector (classification) arranged along the depth dimension.

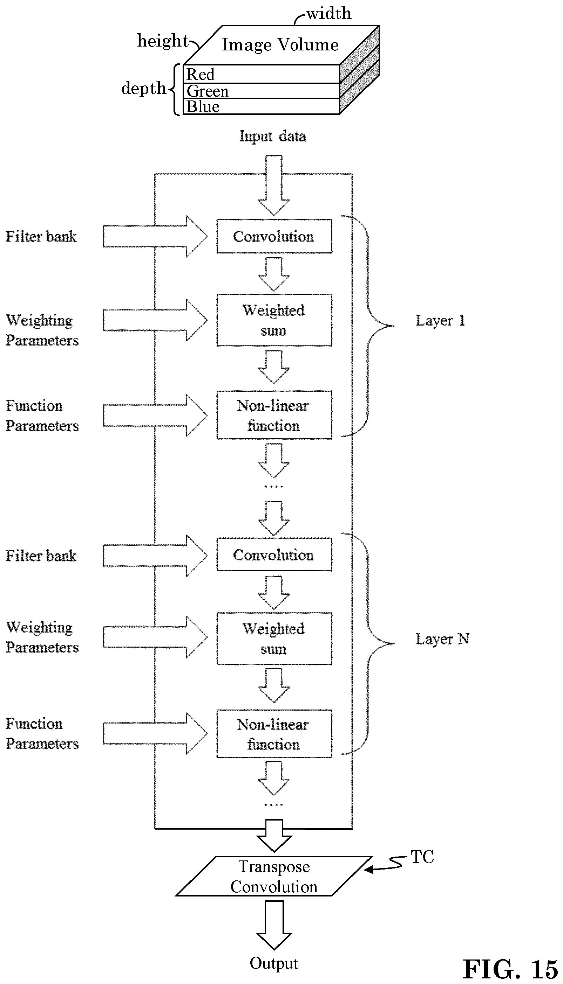

**FIG. 15** provides an example convolutional neural network architecture. A convolutional neural network may be defined as a sequence of two or more layers (e.g., Layer 1 to Layer N), where a layer may include a (image) convolution step, a weighted sum (of results) step, and a non-linear function step. The convolution may be performed on its input data by applying a filter (or kernel), e.g. on a moving window across the input data, to produce a feature map. Each layer and component of a layer may have different pre-determined filters (from a filter bank), weights (or weighting parameters), and/or function parameters. In the present example, the input data is an image, which may be raw pixel values of the image, of a given pixel height and width. In the present example, the input image is illustrated as having a depth of three color channels RGB (Red, Green, and Blue). Optionally, the input image may undergo various preprocessing, and the preprocessing results may be input in place of, or in addition to, the raw input image. Some examples of image preprocessing may include: retina blood vessel map segmentation, color space conversion, adaptive histogram equalization, connected components generation, etc. Within a layer, a dot product may be computed between the given weights and a small region they are connected to in the input volume. Many ways of configuring a CNN are known in the art, but as an example, a layer may be configured to apply an elementwise activation function, such as max (0,x) thresholding at zero. A pooling function may be performed (e.g., along the x-y directions) to down-sample a volume. A fully-connected layer may be used to determine the classification output and produce a one-dimensional output vector, which has been found useful for image recognition and classification. However, for image segmentation, the CNN would need to classify each pixel. Since each CNN layers tends to reduce the resolution of the input image, another stage is needed to up-sample the image back to its original resolution. This may be achieved by application of a transpose convolution (or deconvolution) stage TC, which typically does not use any predefine interpolation method, and instead has learnable parameters.

Convolutional Neural Networks have been successfully applied to many computer vision problems. As explained above, training a CNN generally requires a large training dataset. The U-Net architecture is based on CNNs and can generally can be trained on a smaller training dataset than conventional CNNs.

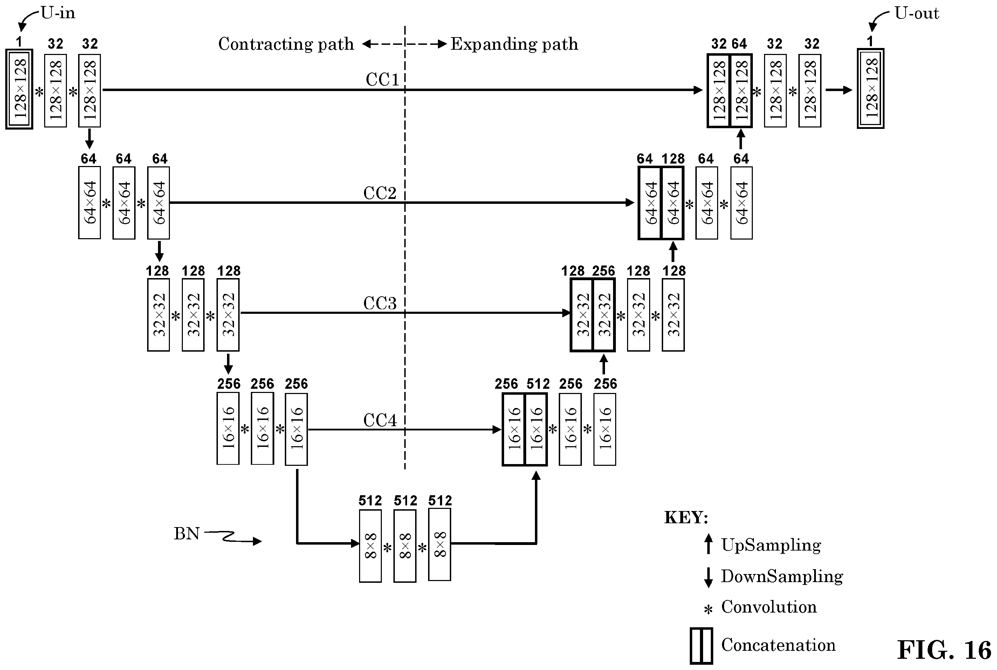

**FIG. 16** illustrates an example U-Net architecture. The present exemplary U-Net includes an input module (or input layer or stage) that receives an input U-in (e.g., input image or image patch) of any given size. For illustration purposes, the image size at any stage, or layer, is indicated within a box that represents the image, e.g., the input module encloses number "128×128" to indicate that input image U-in is comprised of 128 by 128 pixels. The input image may be a fundus image, an OCT/OCTA *en face,* B-scan image, etc. It is to be understood, however, that the input may be of any size or dimension. For example, the input image may be an RGB color image, monochrome image, volume image, etc. The input image undergoes a series of processing layers, each of which is illustrated with exemplary sizes, but these sizes are illustration purposes only and would depend, for example, upon the size of the image, convolution filter, and/or pooling stages. The present architecture consists of a contracting path (herein illustratively comprised of four encoding modules) followed by an expanding path (herein illustratively comprised of four decoding modules), and copy-and-crop links (e.g., CC1 to CC4) between corresponding modules/stages that copy the output of one encoding module in the contracting path and concatenates it to (e.g., appends it to the back of) the up-converted input of a correspond decoding module in the expanding path. This results in a characteristic U-shape, from which the architecture draws its name. Optionally, such as for computational considerations, a "bottleneck" module/stage (BN) may be positioned between the contracting path and the expanding path. The bottleneck BN may consist of two convolutional layers (with batch normalization and optional dropout).

The contracting path is similar to an encoder, and generally captures context (or feature) information by the use of feature maps. In the present example, each encoding module in the contracting path may include two or more convolutional layers, illustratively indicated by an asterisk symbol "*", and which may be followed by a max pooling layer (e.g., DownSampling layer). For example, input image U-in is illustratively shown to undergo two convolution layers, each with 32 feature maps. As it would be understood, each convolution kernel produces a feature map (e.g., the output from a convolution operation with a given kernel is an image typically termed a "feature map"). For example, input U-in undergoes a first convolution that applies 32 convolution kernels (not shown) to produce an output consisting of 32 respective feature maps. However, as it is known in the art, the number of feature maps produced by a convolution operation may be adjusted (up or down). For example, the number of feature maps may be reduced by averaging groups of feature maps, dropping some feature maps, or other known method of feature map reduction. In the present example, this first convolution is followed by a second convolution whose output is limited to 32 feature maps. Another way to envision feature maps may be to think of the output of a convolution layer as a 3D image whose 2D dimension is given by the listed X-Y planar pixel dimension (e.g., 128×128 pixels), and whose depth is given by the number of feature maps (e.g., 32 planar images deep). Following this analogy, the output of the second convolution (e.g., the output of the first encoding module in the contracting path) may be described as a 128×128×32 image. The output from the second convolution then undergoes a pooling operation, which reduces the 2D dimension of each feature map (e.g., the X and Y dimensions may each be reduced by half). The pooling operation may be embodied within the DownSampling operation, as indicated by a downward arrow. Several pooling methods, such as max pooling, are known in the art and the specific pooling method is not critical to the present invention. The number of feature maps may double at each pooling, starting with 32 feature maps in the first encoding module (or block), 64 in the second encoding module, and so on. The contracting path thus forms a convolutional network consisting of multiple encoding modules (or stages or blocks). As is typical of convolutional networks, each encoding module may provide at least one convolution stage followed by an activation function (e.g., a rectified linear unit (ReLU) or sigmoid layer), not shown, and a max pooling operation. Generally, an activation function introduces non-linearity into a layer (e.g., to help avoid overfitting issues), receives the results of a layer, and determines whether to "activate" the output (e.g., determines whether the value of a given node meets predefined criteria to have an output forwarded to a next layer/node). In summary, the contracting path generally reduces spatial information while increasing feature information.

The expanding path is similar to a decoder, and among other things, may provide localization and spatial information for the results of the contracting path, despite the down sampling and any max-pooling performed in the contracting stage. The expanding path includes multiple decoding modules, where each decoding module concatenates its current up-converted input with the output of a corresponding encoding module. In this manner, feature and spatial information are combined in the expanding path through a sequence of up-convolutions (e.g., UpSampling or transpose convolutions or deconvolutions) and concatenations with high-resolution features from the contracting path (e.g., via CC1 to CC4). Thus, the output of a deconvolution layer is concatenated with the corresponding (optionally cropped) feature map from the contracting path, followed by two convolutional layers and activation function (with optional batch normalization). The output from the last expanding module in the expanding path may be fed to another processing/training block or layer, such as a classifier block, that may be trained along with the U-Net architecture.

### Computing Device/System

**FIG. 17** illustrates an example computer system (or computing device or computer device). In some embodiments, one or more computer systems may provide the functionality described or illustrated herein and/or perform one or more steps of one or more methods described or illustrated herein. The computer system may take any suitable physical form. For example, the computer system may be an embedded computer system, a system-on-chip (SOC), a single-board computer system (SBC) (such as, for example, a computer-on-module (COM) or system-on-module (SOM)), a desktop computer system, a laptop or notebook computer system, a mesh of computer systems, a mobile telephone, a personal digital assistant (PDA), a server, a tablet computer system, an augmented/virtual reality device, or a combination of two or more of these. Where appropriate, the computer system may reside in a cloud, which may include one or more cloud components in one or more networks.

In some embodiments, the computer system may include a processor Cpnt1, memory Cpnt2, storage Cpnt3, an input/output (I/O) interface Cpnt4, a communication interface Cpnt5, and a bus Cpnt6. The computer system may optionally also include a display Cpnt7, such as a computer monitor or screen.

Processor Cpnt1 includes hardware for executing instructions, such as those making up a computer program. For example, processor Cpnt1 may be a central processing unit (CPU) or a general-purpose computing on graphics processing unit (GPGPU). Processor Cpnt1 may retrieve (or fetch) the instructions from an internal register, an internal cache, memory Cpnt2, or storage Cpnt3, decode and execute the instructions, and write one or more results to an internal register, an internal cache, memory Cpnt2, or storage Cpnt3. In particular embodiments, processor Cpnt1 may include one or more internal caches for data, instructions, or addresses. Processor Cpnt1 may include one or more instruction caches, one or more data caches, such as to hold data tables. Instructions in the instruction caches may be copies of instructions in memory Cpnt2 or storage Cpnt3, and the instruction caches may speed up retrieval of those instructions by processor Cpnt1. Processor Cpnt1 may include any suitable number of internal registers, and may include one or more arithmetic logic units (ALUs). Processor Cpnt1 may be a multi-core processor; or include one or more processors Cpnt1. Although this disclosure describes and illustrates a particular processor, this disclosure contemplates any suitable processor.

Memory Cpnt2 may include main memory for storing instructions for processor Cpnt1 to execute or to hold interim data during processing. For example, the computer system may load instructions or data (e.g., data tables) from storage Cpnt3 or from another source (such as another computer system) to memory Cpnt2. Processor Cpnt1 may load the instructions and data from memory Cpnt2 to one or more internal register or internal cache. To execute the instructions, processor Cpnt1 may retrieve and decode the instructions from the internal register or internal cache. During or after execution of the instructions, processor Cpnt1 may write one or more results (which may be intermediate or final results) to the internal register, internal cache, memory Cpnt2 or storage Cpnt3. Bus Cpnt6 may include one or more memory buses (which may each include an address bus and a data bus) and may couple processor Cpnt1 to memory Cpnt2 and/or storage Cpnt3. Optionally, one or more memory management unit (MMU) facilitate data transfers between processor Cpnt1 and memory Cpnt2. Memory Cpnt2 (which may be fast, volatile memory) may include random access memory (RAM), such as dynamic RAM (DRAM) or static RAM (SRAM). Storage Cpnt3 may include long-term or mass storage for data or instructions. Storage Cpnt3 may be internal or external to the computer system, and include one or more of a disk drive (e.g., hard-disk drive, HDD, or solid-state drive, SSD), flash memory, ROM, EPROM, optical disc, magneto-optical disc, magnetic tape, Universal Serial Bus (USB)-accessible drive, or other type of non-volatile memory.

I/O interface Cpnt4 may be software, hardware, or a combination of both, and include one or more interfaces (e.g., serial or parallel communication ports) for communication with I/O devices, which may enable communication with a person (e.g., user). For example, I/O devices may include a keyboard, keypad, microphone, monitor, mouse, printer, scanner, speaker, still camera, stylus, tablet, touch screen, trackball, video camera, another suitable I/O device, or a combination of two or more of these.

Communication interface Cpnt5 may provide network interfaces for communication with other systems or networks. Communication interface Cpnt5 may include a Bluetooth interface or other type of packet-based communication. For example, communication interface Cpnt5 may include a network interface controller (NIC) and/or a wireless NIC or a wireless adapter for communicating with a wireless network. Communication interface Cpnt5 may provide communication with a WI-FI network, an ad hoc network, a personal area network (PAN), a wireless PAN (e.g., a Bluetooth WPAN), a local area network (LAN), a wide area network (WAN), a metropolitan area network (MAN), a cellular telephone network (such as, for example, a Global System for Mobile Communications (GSM) network), the Internet, or a combination of two or more of these.

Bus Cpnt6 may provide a communication link between the above-mentioned components of the computing system. For example, bus Cpnt6 may include an Accelerated Graphics Port (AGP) or other graphics bus, an Enhanced Industry Standard Architecture (EISA) bus, a front-side bus (FSB), a HyperTransport (HT) interconnect, an Industry Standard Architecture (ISA) bus, an InfiniBand bus, a low-pin-count (LPC) bus, a memory bus, a Micro Channel Architecture (MCA) bus, a Peripheral Component Interconnect (PCI) bus, a PCI-Express (PCIe) bus, a serial advanced technology attachment (SATA) bus, a Video Electronics Standards Association local (VLB) bus, or other suitable bus or a combination of two or more of these.

Although this disclosure describes and illustrates a particular computer system having a particular number of particular components in a particular arrangement, this disclosure contemplates any suitable computer system having any suitable number of any suitable components in any suitable arrangement.

Herein, a computer-readable non-transitory storage medium or media may include one or more semiconductor-based or other integrated circuits (ICs) (such, as for example, field-programmable gate arrays (FPGAs) or application-specific ICs (ASICs)), hard disk drives (HDDs), hybrid hard drives (HHDs), optical discs, optical disc drives (ODDs), magneto-optical discs, magneto-optical drives, floppy diskettes, floppy disk drives (FDDs), magnetic tapes, solid-state drives (SSDs), RAM-drives, SECURE DIGITAL cards or drives, any other suitable computer-readable non-transitory storage media, or any suitable combination of two or more of these, where appropriate. A computer-readable non-transitory storage medium may be volatile, non-volatile, or a combination of volatile and non-volatile, where appropriate.

While the invention has been described in conjunction with several specific embodiments, it is evident to those skilled in the art that many further alternatives, modifications, and variations will be apparent in light of the foregoing description. Thus, the invention described herein is intended to embrace all such alternatives, modifications, applications and variations as may fall within the scope of the appended claims.

## Claims

1. A method for customizing visual field tests, comprising:
selecting a visual field test for a patient, the selected visual field test having one or more test points of definable light intensities;
obtaining a biometric measurement of a retina of the patient;
deriving a respective threshold sensitivity value for one or more select test points of the selected visual field test based at least in part on the biometric measurement, each threshold sensitivity value being a light intensity measure that the patient is expected to discern with a predefined success rate; and
using the derived threshold sensitivity values to determine one or more starting intensity values for the one or more select test points when applying the selected visual field test to the patient; **characterised in that** the deriving is provided at least in part by a
machine learning system established, at least in part, within a computing system including a trained neural network that includes a
first neural network of a first type (NN-1) in series with a second neural network (NN-2) different than the first type, and wherein the first type neural network receives as input image data and the second type neural network receives as input non-image data.

2. The method of claim 1, wherein:
the biometric measurement is based at least in part on an image of the retina;
the image of the retina is captured by a specific imaging device using a specific imaging modality; and
the imaging modality is preferably one of grayscale, color, infrared, retinal layer thickness map, optical coherence tomography (OCT), Doppler OCT, and fluorescein angiography.

3. The method of claim 2, wherein the imaging modality is fundus imaging and the image of the retina is a fundus image, or
wherein the image of the retina is an optical coherence tomography (OCT) angiography image.

4. The method of any of claims 1 to 3, wherein training of the neural network includes:
collecting a plurality of training data pairs, each training data pair including training input data and corresponding training output data, the training input data including a biometric measurement of a retina of a patient, and the training output data including a test result from a specific visual field test given to the patient;
for each training data pair, submitting its training input data as input to the neural network and providing as target output from the neural network its corresponding visual field test result;
wherein the training input data further includes one or more of a physical feature of the patient, normative biometric data of a demographic of the patient, and normative functional data of the demography of the patient; and
the physical feature includes one or more of the patient's age, ethnic group, and medical history;
the normative biometric data includes one or more of a retinal nerve fiber layer (RNFL) thickness and a ganglion cell-inner plexiform layer (GCIPL) thickness for the demographic of the patient; and
the normative functional data includes one or more standardized initialization parameter of the specific visual field test for the demographic of the patient.

5. The method of claim 4, wherein the biometric measurement includes an OCT scan of the retina of the patient.

6. The method of one or more of claims 1 to 5, wherein the first and second neural network are of types selected from a fully-connected neural network, convolutional neural network, feedforward neural network, recurrent neural network, modular neural network, and U-Net.

7. The method of one or more of claims 1 to 6, wherein the selected visual field test is one of a static automated perimetry test, a kinetic perimetry test, and a frequency doubling perimetry test.

8. The method of one or more of claims 1 to 7, wherein the deriving of a respective threshold sensitivity value for one or more select test points of the selected visual field test is further based on at least one of the patient's previous true visual field test results and the patient's previously derived visual field test prediction that is itself based on a historical biometric measurement of the retina of the patient taken on a previous date than the currently obtained biometric measurement of the retina of the patient.

9. A system for customizing a functional visual field test, comprising:
an electronic processor (Cpnt1);
a perimeter (13) configured to apply the visual field test to a patient, the visual field test having one or more test points of definable light intensities;
a non-transitory computer readable storage device (Cpnt3) storing software instructions that, when executed by the processor, cause the electronic processor to:
obtain a biometric measurement of a retina of the patient; and
determine a respective threshold sensitivity value for one or more select test points of the visual field test based at least in part on the biometric measurement, each threshold sensitivity value being a light intensity measure that the patient is expected to discern with a predefined success rate;
wherein the perimeter (13) is configured to use
the determined threshold sensitivity values to
determine starting intensity values for the one or more select test points when applying the visual field test to the patient;
**characterised in that**
the electronic processor (Cpnt1) is part of a neural network that includes a first neural network of a first type (NN-1) in series with a second neural network (NN-2) different than the first type, and **in that** the first type neural network is configured to receive as input image data and the second type neural network is configured to receive as input non-image data.

10. The system of claim 9, wherein the biometric measurement is based at least in part on a fundus image of the retina acquired with fundus photography.

11. The system of claim 9 or 10, wherein training of the trained neural network includes:
collecting a plurality of training data pairs, each training data pair including training input data and corresponding training output data, the training input data including a biometric measurement of a retina of a patient, and the training output data including a test result from a specific visual field test given to the patient;
for each training data pair, submitting its training input data as input to the neural network and providing as target output from the neural network its corresponding visual field test result;
wherein the training input data further includes one or more of a physical feature of the patient, normative biometric data of a demographic of the patient, and normative functional data of the demography of the patient; and
the physical feature includes one or more of the patient's age, ethnic group, and medical history;
the normative biometric data includes one or more of a retinal nerve fiber layer (RNFL) thickness and a ganglion cell-inner plexiform layer (GCIPL) thickness for the demographic of the patient; and
the normative functional data includes one or more standardized initialization parameter of the specific visual field test for the demographic of the patient.

12. The system of claim 11, wherein the biometric measurement of a retina of the patient includes an OCT angiography scan of the retina of the patient.

13. The system of one or more of claims 9 to 12, wherein the first and second neural network are selected from of a fully-connected neural network, convolutional neural network, feedforward neural network, recurrent neural network, modular neural network, and U-Net.

14. The system of one or more of claims 11 to 13, wherein the visual field test is one of a static automated perimetry test, a kinetic perimetry test, and a frequency doubling perimetry test.

15. The system of one or more of claims 9 to 14, wherein the determining of a respective threshold sensitivity value for one or more select test points of the selected visual field test is further based on at least one of the patient's previous true visual field test results and the patient's previously determined threshold sensitivity value that is itself based on a historical biometric measurement of the retina of the patient taken on a previous date than the currently obtained biometric measurement of the retina of the patient.

## Patentansprüche

1. Verfahren zum Maßschneidern von Gesichtsfeldtests, umfassend:
Auswählen eines Gesichtsfeldtests für einen Patienten, wobei der ausgewählte Gesichtsfeldtest einen oder mehrere Testpunkte von definierbaren Lichtintensitäten aufweist;
Erhalten einer biometrischen Messung von einer Retina des Patienten;
Ableiten eines jeweiligen Schwellenempfindlichkeitswerts für einen oder mehrere ausgewählte Testpunkte des ausgewählten Gesichtsfeldtests zumindest teilweise auf der Grundlage der biometrischen Messung, wobei jeder Schwellenempfindlichkeitswert ein Lichtintensitätsmaß ist, bei dem man vom Patienten erwartet, dass dieses mit einer vordefinierten Erfolgsrate unterschieden wird; und
unter Verwendung der abgeleiteten Schwellenempfindlichkeitswerte, zum Bestimmen eines oder mehrerer Startintensitätswerte für den einen oder die mehreren ausgewählten Testpunkte, wenn der ausgewählte Gesichtsfeldtest auf den Patienten angewandt wird;
**dadurch gekennzeichnet, dass** das Ableiten zumindest teilweise durch ein Maschinenlernsystem bereitgestellt wird, das, zumindest teilweise, innerhalb eines Rechensystems erstellt ist, das ein trainiertes neuronales Netz beinhaltet, das ein erstes neuronales Netz einer ersten Art (NN-1) in Serie mit einem zweiten neuronalen Netz (NN-2), von anderer als der ersten Art, beinhaltet; und wobei das neuronale Netz der ersten Art als Eingabe Bilddaten empfängt und das neuronale Netz der zweiten Art als Eingabe Nicht-Bilddaten empfängt.

2. Verfahren nach Anspruch 1, wobei:
die biometrische Messung zumindest teilweise auf einem Bild der Retina basiert;
das Bild der Retina unter Verwendung einer spezifischen Bildgebungsmodalität von einer spezifischen Bildgebungsvorrichtung aufgenommen wird, und
die Bildgebungsmodalität bevorzugt eine von Grauwert, Farbe, Infrarot, Retinaschichtdickenkarte, optischer Kohärenztomographie (OCT), Doppler-OCT und Fluoreszin-Angiographie ist.

3. Verfahren nach Anspruch 2, wobei die Bildgebungsmodalität Fundusbildgebung ist und das Bild der Retina ein Fundusbild ist, oder
wobei das Bild der Retina ein Bild aus optischer Kohärenztomographie(OCT)-Angiographie ist.

4. Verfahren nach einem der Ansprüche 1 bis 3, wobei Trainieren des neuronalen Netzes Folgendes beinhaltet:
Sammeln einer Vielzahl von Trainingsdatenpaaren, wobei jedes Trainingsdatenpaar Trainingseingabedaten und korrespondierende Trainingsausgabedaten beinhaltet, wobei die Trainingseingabedaten eine biometrische Messung einer Retina eines Patienten beinhalten, und wobei die Trainingsausgabedaten ein Testresultat von einem spezifischen Gesichtsfeldtest, dem der Patient unterzogen wurde, beinhalten;
für jedes Trainingsdatenpaar, Übergeben von dessen Trainingseingabedaten als Eingabe an das neuronale Netz und Liefern, als Zielausgabe aus dem neuronalen Netz, dessen korrespondierendes Gesichtsfeldtestresultat;
wobei die Trainingseingabedaten ferner eines oder mehrere von einem physischen Merkmal des Patienten, normative biometrische Daten einer Demographie des Patienten und normative funktionale Daten der Demographie des Patienten beinhalten und
wobei das physische Merkmal eines oder mehrere von dem Alter, der ethnischen Gruppe und der Anamnese des Patienten beinhaltet;
wobei die normativen biometrischen Daten eines oder mehrere von Dicke der Retinanervenfaserschicht bzw. RNFL-Dicke und Dicke einer Ganglionzellen-Inneren-Plexiformen-Schicht bzw. GCIPL-Dicke für die Demographie des Patienten beinhalten; und
wobei die normativen funktionalen Daten einen oder mehrere standardmäßige Initialisierungsparameter des spezifischen Gesichtsfeldtests für die Demographie des Patienten beinhalten.

5. Verfahren nach Anspruch 4, wobei die biometrische Messung einen OCT-Scan der Retina des Patienten beinhaltet.

6. Verfahren nach einem oder mehreren der Ansprüche 1 bis 5, wobei das erste und das zweite neuronale Netz von Arten sind, die ausgewählt werden aus einem vollverbundenen neuronalen Netz, einem neuronalen Faltungsnetz, einem vorwärtsgekoppelten neuronalen Netz, einem rekurrenten neuronalen Netz, einem modularen neuronalen Netz und einem U-Net.

7. Verfahren nach einem oder mehreren der Ansprüche 1 bis 6, wobei der ausgewählte Gesichtsfeldtest einer von einem statischen automatisierten Perimetrietest, einem kinetischen Perimetrietest und einem frequenzverdoppelnden Perimetrietest ist.

8. Verfahren nach einem oder mehreren der Ansprüche 1 bis 7, wobei das Ableiten eines jeweiligen Schwellenempfindlichkeitswerts für einen oder mehrere ausgewählte Testpunkte des ausgewählten Gesichtsfeldtests ferner basiert auf zumindest einem von vorherigen wahren Gesichtsfeldtestresultaten des Patienten und der zuvor abgeleiteten Gesichtsfeldtestvorhersage des Patienten, die selbst auf historischen biometrischen Messungen der Retina des Patienten basiert, die an einem früheren Datum aufgenommen wurden als die erhaltenen aktuellen biometrischen Messungen der Retina des Patienten.

9. System zum Maßschneidern eines Funktional-Gesichtsfeldtests, umfassend:
einen elektronischen Prozessor (Cpnt1);
ein Perimeter (13), ausgelegt zum Anwenden des Gesichtsfeldtests auf einen Patienten, wobei der Gesichtsfeldtest einen oder mehrere Testpunkte von definierbaren Lichtintensitäten aufweist;
eine nicht-transitorische computerlesbare Speicherungsvorrichtung (Cpnt3), die Softwareanweisungen speichert, die bei Ausführung durch den Prozessor den elektronischen Prozessor veranlassen zum:
Erhalten einer biometrischen Messung von einer Retina des Patienten; und
Bestimmen eines jeweiligen Schwellenempfindlichkeitswerts für einen oder mehrere ausgewählte Testpunkte des Gesichtsfeldtests zumindest teilweise auf der Grundlage der biometrischen Messung, wobei jeder Schwellenempfindlichkeitswert ein Lichtintensitätsmaß ist, bei dem man vom Patienten erwartet, dass dieses mit einer vordefinierten Erfolgsrate unterschieden wird;
wobei das Perimeter (13) ausgelegt ist zum Verwenden der bestimmten Schwellenempfindlichkeitswerte zum Bestimmen von Startintensitätswerten für den einen oder die mehreren ausgewählten Testpunkte, wenn der Gesichtsfeldtest auf den Patienten angewandt wird;
**dadurch gekennzeichnet, dass**
der elektronische Prozessor (Cpnt1) Teil eines trainierten neuronalen Netzes ist, das ein erstes neuronales Netz einer ersten Art (NN-1) in Serie mit einem zweiten neuronalen Netz (NN-2), von anderer als der ersten Art, beinhaltet; und dass das neuronale Netz der ersten Art ausgelegt ist zum Empfangen von Bilddaten als Eingabe und das neuronale Netz der zweiten Art ausgelegt ist zum Empfangen von Nicht-Bilddaten als Eingabe.

10. System nach Anspruch 9, wobei die biometrische Messung zumindest teilweise auf einem Fundusbild der Retina basiert, das mit Fundusfotografie erfasst wurde.

11. System nach Anspruch 9 oder 10, wobei Trainieren des trainierten neuronalen Netzes Folgendes beinhaltet:
Sammeln einer Vielzahl von Trainingsdatenpaaren, wobei jedes Trainingsdatenpaar Trainingseingabedaten und korrespondierende Trainingsausgabedaten beinhaltet, wobei die Trainingseingabedaten eine biometrische Messung von einer Retina eines Patienten beinhalten, und wobei die Trainingsausgabedaten ein Testresultat von einem spezifischen Gesichtsfeldtest, dem der Patient unterzogen wurde, beinhalten;
für jedes Trainingsdatenpaar, Übergeben von dessen Trainingseingabedaten als Eingabe an das neuronale Netz und Liefern, als Zielausgabe aus dem neuronalen Netz, dessen korrespondierendes Gesichtsfeldtestresultat;
wobei die Trainingseingabedaten ferner eines oder mehrere von einem physischen Merkmal des Patienten, normative biometrische Daten einer Demographie des Patienten und normative funktionale Daten der Demographie des Patienten beinhalten und
wobei das physische Merkmal eines oder mehrere von dem Alter, der ethnischen Gruppe und der Anamnese des Patienten beinhaltet;
wobei die normativen biometrischen Daten eines oder mehrere von Dicke der Retinanervenfaserschicht bzw. RNFL-Dicke und Dicke einer Ganglionzellen-Inneren-Plexiformen-Schicht bzw. GCIPL-Dicke für die Demographie des Patienten beinhalten; und
wobei die normativen funktionalen Daten einen oder mehrere standardmäßige Initialisierungsparameter des spezifischen Gesichtsfeldtests für die Demographie des Patienten beinhalten.

12. System nach Anspruch 11, wobei die biometrische Messung einer Retina des Patienten einen OCT-Angiographie-Scan der Retina des Patienten beinhaltet.

13. System nach einem oder mehreren der Ansprüche 9 bis 12, wobei das erste und das zweite neuronale Netz ausgewählt werden aus einem vollverbundenen neuronalen Netz, einem neuronalen Faltungsnetz, einem vorwärtsgekoppeltem neuronalen Netz, einem rekurrenten neuronalen Netz, einem modularen neuronalen Netz und einem U-Net.

14. System nach einem oder mehreren der Ansprüche 11 bis 13, wobei der Gesichtsfeldtest einer von einem statischen automatisierten Perimetrietest, einem kinetischen Perimetrietest und einem frequenzverdoppelnden Perimetrietest ist.

15. System nach einem oder mehreren der Ansprüche 9 bis 14, wobei das Bestimmen eines jeweiligen Schwellenempfindlichkeitswerts für einen oder mehrere ausgewählte Testpunkte der ausgewählten Gesichtsfeldtests ferner basiert auf zumindest einem von vorherigen wahren Gesichtsfeldtestresultaten des Patienten und dem zuvor bestimmten Schwellenempfindlichkeitswert des Patienten, der selbst auf historischen biometrischen Messungen der Retina des Patienten basiert, die an einem früheren Datum aufgenommen wurden als die erhaltenen aktuellen biometrischen Messungen der Retina des Patienten.

## Revendications

1. Procédé pour personnaliser des tests du champ visuel, le procédé comprenant les étapes suivantes :
sélectionner un test du champ visuel pour un patient, le test du champ visuel sélectionné ayant un ou plusieurs points de test d'intensités lumineuses définissables ;
obtenir une mesure biométrique d'une rétine du patient ; dériver une valeur de sensibilité seuil respective pour un ou plusieurs points de test sélectionnés du test du champ visuel sélectionné, sur la base, au moins en partie, de la mesure biométrique, chaque valeur de sensibilité seuil étant une mesure d'intensité lumineuse que le patient est censé discerner avec un taux de réussite prédéfini ; et
utiliser les valeurs de sensibilité seuil dérivées pour déterminer une ou plusieurs valeurs d'intensité de départ pour les un ou plusieurs points de test sélectionnés lors de l'application du test du champ visuel sélectionné au patient ;
**caractérisé en ce que** la dérivation est fournie au moins en partie par un système d'apprentissage automatique établi, au moins en partie, au sein d'un système informatique comprenant un réseau neuronal entraîné qui comprend un premier réseau neuronal d'un premier type (NN-1) en série avec un deuxième réseau neuronal (NN-2) différent de celui du premier type, et où le réseau neuronal du premier type reçoit en entrée des données d'image et le réseau neuronal du deuxième type reçoit en entrée des données non d'image.

2. Procédé selon la revendication 1, dans lequel :
la mesure biométrique est basée au moins en partie sur une image de la rétine ;
l'image de la rétine est capturée par un dispositif d'imagerie spécifique utilisant une modalité d'imagerie spécifique ; et
la modalité d'imagerie est de préférence l'une des modalités suivantes : échelle de gris, couleur, infrarouge, carte d'épaisseur de la couche rétinienne, tomographie par cohérence optique (OCT), OCT Doppler et angiographie à la fluorescéine.

3. Procédé selon la revendication 2, dans lequel la modalité d'imagerie est l'imagerie du fond de l'œil et l'image de la rétine est une image du fond de l'œil, ou dans lequel l'image de la rétine est une image d'angiographie par tomographie par cohérence optique (OCT) .

4. Procédé selon l'une quelconque des revendications 1 à 3, dans lequel l'apprentissage du réseau neuronal comprend les étapes suivantes :
collecter une pluralité de paires de données d'apprentissage, chaque paire de données d'apprentissage comprenant des données d'entrée d'apprentissage et des données de sortie d'apprentissage correspondantes, les données d'entrée d'apprentissage comprenant une mesure biométrique d'une rétine d'un patient, et les données de sortie d'apprentissage comprenant un résultat de test provenant d'un test de champ visuel spécifique réalisé sur le patient ;
pour chaque paire de données d'apprentissage, soumettre ses données d'entrée d'apprentissage en tant qu'entrée au réseau neuronal et fournir, en tant que sortie cible du réseau neuronal, son résultat de test de champ visuel correspondant ;
où les données d'entrée d'apprentissage comprennent en outre un ou plusieurs éléments parmi une caractéristique physique du patient, des données biométriques normatives de données démographiques du patient et des données fonctionnelles normatives de la démographie du patient ; et
la caractéristique physique comprend une ou plusieurs caractéristiques parmi l'âge, le groupe ethnique et les antécédents médicaux du patient ;
les données biométriques normatives comprennent une ou plusieurs des données suivantes : l'épaisseur de la couche de fibres nerveuses rétiniennes (RNFL) et
l'épaisseur de la couche des cellules ganglionnaires et du plexiforme interne (GCIPL) pour le groupe démographique du patient ; et
les données fonctionnelles normatives comprennent un ou plusieurs paramètres d'initialisation normalisés du test de champ visuel spécifique pour le groupe démographique du patient.

5. Procédé selon la revendication 4, dans lequel la mesure biométrique comprend un balayage OCT de la rétine du patient.

6. Procédé selon une ou plusieurs des revendications 1 à 5, dans lequel les premier et deuxième réseaux neuronaux sont de types choisis parmi un réseau neuronal entièrement connecté, un réseau neuronal convolutif, un réseau neuronal à action directe, un réseau neuronal récurrent, un réseau neuronal modulaire et un réseau U-Net.

7. Procédé selon une ou plusieurs des revendications 1 à 6, dans lequel le test du champ visuel sélectionné est un test parmi un test de périmétrie automatisée statique, un test de périmétrie cinétique ou un test de périmétrie à doublement de fréquence.

8. Procédé selon une ou plusieurs des revendications 1 à 7, dans lequel la dérivation d'une valeur de sensibilité seuil respective pour un ou plusieurs points de test sélectionnés du test du champ visuel sélectionné est en outre basée sur au moins l'un des éléments suivants : les résultats de tests du champ visuel précédents du patient et la prédiction du test du champ visuel précédemment dérivée du patient qui est elle-même basée sur une mesure biométrique historique de la rétine du patient prise à une date antérieure à la mesure biométrique de la rétine du patient obtenue actuellement.

9. Système pour personnaliser un test fonctionnel du champ visuel, comprenant :
un processeur électronique (Cpnt1) ;
un périmètre (13) configuré pour appliquer le test du champ visuel à un patient, le test du champ visuel comprenant un ou plusieurs points de test d'intensités lumineuses définissables ;
un dispositif de stockage non transitoire lisible par ordinateur (Cpnt3) stockant des instructions logicielles qui, lorsqu'elles sont exécutées par le processeur, amènent le processeur électronique à :
obtenir une mesure biométrique d'une rétine du patient ; et
déterminer une valeur de sensibilité seuil respective pour un ou plusieurs points de test sélectionnés du test du champ visuel sur la base, au moins en partie, de la mesure biométrique, chaque valeur de sensibilité seuil étant une mesure d'intensité lumineuse que le patient est censé discerner avec un taux de réussite prédéfini ;
où le périmètre (13) est configuré pour utiliser les valeurs de sensibilité seuil déterminées afin de déterminer des valeurs d'intensité de départ pour les un ou plusieurs points de test sélectionnés lors de l'application du test du champ visuel au patient ;
**caractérisé en ce que**
le processeur électronique (Cpnt1) fait partie d'un réseau neuronal qui comprend un premier réseau neuronal d'un premier type (NN-1) en série avec un deuxième réseau neuronal (NN-2) différent de celui du premier type, et **en ce que** le réseau neuronal du premier type est configuré pour recevoir en entrée des données d'image et le réseau neuronal du deuxième type est configuré pour recevoir en entrée des données non d'image.

10. Système selon la revendication 9, dans lequel la mesure biométrique est basée au moins en partie sur une image du fond de l'œil de la rétine acquise par photographie du fond de l'œil.

11. Système selon la revendication 9 ou la revendication 10, dans lequel l'apprentissage du réseau neuronal entraîné comprend les étapes suivantes :
collecter une pluralité de paires de données d'apprentissage, chaque paire de données d'apprentissage comprenant des données d'entrée d'apprentissage et des données de sortie d'apprentissage correspondantes, les données d'entrée d'apprentissage comprenant une mesure biométrique d'une rétine d'un patient, et les données de sortie d'apprentissage comprenant un résultat de test provenant d'un test de champ visuel spécifique réalisé sur le patient ;
pour chaque paire de données d'apprentissage, soumettre ses données d'entrée d'apprentissage en tant qu'entrée au réseau neuronal et fournir, en tant que sortie cible du réseau neuronal, son résultat de test de champ visuel correspondant ;
où les données d'entrée d'apprentissage comprennent en outre un ou plusieurs éléments parmi une caractéristique physique du patient, des données biométriques normatives de données démographiques du patient et des données fonctionnelles normatives de la démographie du patient ;
et
la caractéristique physique comprend une ou plusieurs caractéristiques parmi l'âge, le groupe ethnique et les antécédents médicaux du patient ;
les données biométriques normatives comprennent une ou plusieurs des données suivantes : l'épaisseur de la couche de fibres nerveuses rétiniennes (RNFL) et l'épaisseur de la couche des cellules ganglionnaires et du plexiforme interne (GCIPL) pour le groupe démographique du patient ; et
les données fonctionnelles normatives comprennent un ou plusieurs paramètres d'initialisation normalisés du test de champ visuel spécifique pour le groupe démographique du patient.

12. Système selon la revendication 11, dans lequel la mesure biométrique d'une rétine du patient comprend un balayage angiographique OCT de la rétine du patient.

13. Système selon une ou plusieurs des revendications 9 à 12, dans lequel les premier et deuxième réseaux neuronaux sont sélectionnés parmi un réseau neuronal entièrement connecté, un réseau neuronal convolutif, un réseau neuronal à action directe, un réseau neuronal récurrent, un réseau neuronal modulaire et un réseau U-Net.

14. Système selon une ou plusieurs des revendications 11 à 13, dans lequel le test du champ visuel est un test parmi un test de périmétrie automatisée statique, un test de périmétrie cinétique ou un test de périmétrie à doublement de fréquence.

15. Système selon une ou plusieurs des revendications 9 à 14, dans lequel la détermination d'une valeur de sensibilité seuil respective pour un ou plusieurs points de test sélectionnés du test du champ visuel sélectionné est en outre basée sur au moins l'un des éléments suivants : les résultats de tests du champ visuel précédents du patient et la valeur de sensibilité seuil précédemment déterminée du patient qui est elle-même basée sur une mesure biométrique historique de la rétine du patient prise à une date antérieure à la mesure biométrique de la rétine du patient obtenue actuellement.
